# EUROPEAN PATENT APPLICATION

(11) **EP 4 756 821 A1**
(43) Date of publication of application: **10.06.2026**
(21) Application number: 25172686.5
(22) Date of filing: 25.04.2025
(51) Int. Cl.: G16H 20/10, A61B 5/00, G16H 50/30, G06Q 30/0226

(54) **SYSTEMS AND METHODS FOR DIGITAL REMOTE DELIVERY OF PERSONALIZED CONTINGENCY MANAGEMENT TO OPTIMIZE INDIVIDUALIZED TREATMENT OF SUBSTANCE USE DISORDERS**

(30) Priority: 09.12.2024 US 202418974197
(71) Applicant: Click Therapeutics, Inc., New York, NY 10013 (US)
(72) Inventor: FORSTER, Sarah, New York, 10013 (US); MILLER, Julie, New York, 10013 (US)
(74) Representative: Marks & Clerk LLP

(57) **Abstract**

Presented herein are systems and methods for managing data structures for remote monitoring and verification of user devices. A computing system may maintain a data structure including an activity field and a verification field, and determine that activity data generated responsive to the user performing an activity via the application satisfies an activity condition. The computing system may update the data structure to include an activity value and receive verification data. The computing system may update the data structure to include a verification value and execute an operation to update the profile using a token based on the activity value and the verification value. The computing system may improve the efficacy of a medication to address a condition in the user.

## Description

### CROSS-REFERENCE TO RELATED APPLICATIONS

The present application claims priority to and the benefit of U.S. Patent Application No. 18/974,197, titled "SYSTEMS AND METHODS FOR DIGITAL REMOTE DELIVERY OF PERSONALIZED CONTINGENCY MANAGEMENT TO OPTIMIZE INDIVIDUALIZED TREATMENT OF SUBSTANCE USE DISORDERS", and filed on December 9, 2024, the entire contents of which are hereby incorporated by reference in entirety.

### BACKGROUND

Substance abuse disorder (SUD) arises due to ongoing, compulsive use of alcohol, drugs, or other such substances leading to cognitive impairment, health risks, disability, and other negative effects. This condition arises from a diverse array of psychological and social factors. For example, specific factors that can lead to SUDs include genetics (e.g., family history of addiction), exposure to substance use (e.g., early exposure and use), stress, trauma, peer pressure, mental health disorders (e.g., post-traumatic stress disorder, depression, anxiety), coping mechanisms, chronic pain, lack of education, co-occurring mental health disorders (e.g., bipolar disorder, borderline personality disorder), or poverty (e.g., limited educational or employment opportunities), among others.

This condition impacts the physical, social, mental, and financial health as well as the overall quality of life of individuals affected. SUD can lead to a range of health complications, such as liver disease, heart issues, or respiratory problems, among others. Additionally, SUD can worsen mental health disorder symptoms and increase a risk of self-harm. The overall quality of life for those with SUD is often severely compromised, reducing satisfaction with life. On the nervous system level, substances alter neurotransmitter levels and cause dependence and increased cravings for substances. Sustained abuse of drugs can lead to structural changes in brain regions such as the prefrontal cortex and also cause cognitive impairment (e.g., memory loss, difficulty concentrating). For various other physical health systems (e.g., cardiovascular, liver), SUD can lead to increased blood pressure and a higher susceptibility to heart disease, arrhythmia, hepatitis, constipation, malnutrition, lung infection, or pulmonary hypertension, among others. SUD may also cause premature aging, such as wrinkles and skin discoloration, weight loss or gain, as well as a decline in dental health, such as tooth decay.

Individuals with SUDs can be treated to wean off their reliance on substances via behavioral conditioning. One example treatment is contingency management (CM), which is a behavioral therapy technique that uses positive reinforcement to encourage individuals to achieve target behaviors (also referred to as operant conditioning intervention). In substance abuse treatment, CM involves providing positive reinforcement (e.g., in the form of rewards) in response to evidence of abstinence. CM operates on the principle that immediate, tangible positive reinforcement strengthens desired behaviors, helping individuals overcome addiction and maintain recovery. At the nervous system level, when an individual receives a reward following a positive behavior, dopamine is released in the user's brain reward system (e.g., the nucleus accumbent). This dopamine release reinforces the behavior by creating a positive association, making it more likely that the individual will repeat the behavior. Over time, the repeated pairing of behavior with positive reinforcements can alter neural circuitry, gradually shifting behavior patterns and reducing reliance on maladaptive behaviors associated with substance use. Traditionally, CM involves face-to-face interactions between individuals and care providers, allowing for immediate feedback.

In some conventional approaches of implementing CM, CM is typically delivered such that the intervention directly targets a specific target behavior. The target behavior may be verified abstinence from targeted substances or completion of additional treatment-related activities. If two broad categories of behaviors are simultaneously targeted, under traditional CM, these behaviors are reinforced through independent reinforcement tracks. In other words, reinforcement of one target behavior is dependent on the individual's history of successfully completing that target behavior on that track. Additionally, the reinforcement of another target behavior is dependent on the individual's history of successfully completing that target behavior on this separate track. Having separate, independent reinforcement tracks is counterproductive and can lead to worse clinical outcomes due to dilution of the effects of reinforcement on the individual, especially if the types of behaviors have differing levels of difficulty.

CM has not been able to be successfully implemented in a digital therapeutic platform due to a variety of challenges faced when attempting to provide a digital CM solution. First, the conventional CM approaches are not personalized to the individual user or dynamically change in response to the user's progress and thus are not as effective. Second, the conventional CM approaches do not directly reinforce abstinence as a primary target behavior. Relatedly, the conventional CM approaches may be seen as direct incentivization of in-app activity completion, leading to regulatory, legal, and optics concerns. Third, the conventional CM approaches dilute abstinence-contingent rewards due to the multiple target behaviors. Fourth, the lag in time caused by conventional CM approaches in receiving the reward does not reinforce the positive behavior change, such as a negative drug test. Fifth, the implementation costs of conventional CM approaches are too high due to the multiple target behaviors. Sixth, the conventional CM approaches with multiple target behaviors are seen as too complex from the user's perspective.

### SUMMARY

To address these and other technical problems with remotely carrying out contingency management for individuals with substance abuse disorder, the digital therapeutic application detailed herein provides for a novel approach to contingency management (CM) by delivering optimal personalized positive reinforcement to best encourage desired behavior to provide recovery and abstinence using individualized support and engagement (RAISE^{™}). There are numerous advantages with the digital therapeutic application as described herein.

First, the digital therapeutic application provides for individualized CM by tailoring reinforcement and reward structures to a particular user's behavior and progress, factoring in multi-dimensional data about the user and their condition. The algorithms for providing positive reinforcement dynamically adjust based on real-time user data gathered through the user's device. This approach ensures that the reinforcement provided is clinically effective, maximizes user engagement, and adapts to the evolving needs of the particular user over time.

Second, the single, integrated primary reward track provided by the digital therapeutic application for the user directly reinforces abstinence by only delivering the reward in the context of verified abstinence. By removing the additional reward track, there is no longer any concern that rewards could be accumulated for activity completion (direct financial incentivization of certain activities) or used to purchase substances, thus offsetting regulatory, legal, and optics concerns. Requiring abstinence verification for rewards further provides a safeguard against this possibility. This is an improvement over conventional approaches on CM that rely on restrictions on certain types of purchases. The digital therapeutic application herein, in contrast, mitigates these risks by ensuring that individualized rewards are always contingent upon abstinence.

Third, rewarding abstinence directly and activity completion indirectly also ensures that the value of rewards associated with the highest priority behavior (namely, abstinence) is not diluted by the completion of other target behaviors. Conventional CM methods that use multiple independent reward tracks, lead to accumulating significant rewards on one track and thus could potentially diminish the value of rewards associated with another track, leading to the reward dilution effect. This may be particularly problematic when one target behavior is easier to accomplish relative to another. For example, completion of an in-app lesson could be considered easier to complete for some users than maintaining abstinence from substances for several days. It would be problematic and counterproductive to encourage abstinence from substances to reward both of these equally. The digital therapeutic application herein addresses this issue by directly reinforcing abstinence and indirectly reinforcing activity completion via individualized abstinence-contingent rewards.

Fourth, because the positive reinforcement is linked closely in time to the target behavior and delivered in a timely fashion to the user through their user device, this can significantly increase the therapeutic impact of the CM. Specifically, by leveraging the ability of remotely monitoring and verifying, the digital therapeutic application herein can reinforce proximal behavior change (e.g., a negative drug test) to support abstinence in a timely manner.

Fifth, the unique combination of direct and indirect reinforcement supports the inclusion of two different target behaviors at a reduced cost relative to independent direct reinforcement of each target behavior separately.

Sixth, a single integrated reward track that incorporates the multidimensional aspects of direct and indirect reinforcement from targeting multiple, different types of behaviors is easier for users to track and understand. For instance, the digital therapeutic application can display feedback in an easy-to-digest manner on progress towards achieving milestones with respect to opportunities for both direct and indirect reinforcement. By contrast, the conventional CM approaches with multiple independent reward tracks can be confusing for users when tracking progress over time.

The limitations with existing CM models are addressed by the combination of the following elements of the digital therapeutic application detailed herein. The first element of the CM delivery of the digital therapeutic application includes a probabilistic reward for positive reinforcement upon completion of recovery activities and verification of abstinence tests. This individualized mechanism allows for indirect incentivization of recovery activity completion via access to a higher-value probabilistic pool of reward upon verification of abstinence within a time window. This allows for reinforcement of treatment adherence and positive behavior change while still requiring verification of abstinence to gain access to the boosted, probabilistic reward opportunities.

The second element includes prize-based reinforcement of verified abstinence. Each reward opportunity is directly contingent upon objective verification of abstinence, such as the receipt of negative results for drug tests. Whether positive reinforcements are drawn from one reward pool (e.g., lower expected values) or another reward pool (e.g., higher expected values) is influenced by dynamic activation of the reinforcement algorithm.

The third element includes remote verification of abstinence from targeted substances. Objective verification of abstinence is conducted remotely to support reliable engagement with digitally-delivered CM. Test results acquired from remote sources are subsequently ingested by the digital therapeutic application to determine opportunities for probabilistic reward delivered for the user.

The fourth element includes remote verification of recovery activity completion. To reinforce indicators of positive changes in behavior, completion of specific recovery activities is also remotely verified. These activities can occur both in-app (e.g., completion of personally tailored therapy lessons on the user's device) or offline behaviors (e.g., verification of activity via monitoring or submission of electronic documentation).

These and other elements of the digital therapeutic application for CM represents a novel, non-conventional solution to the complexities of digital therapeutic applications, especially in the context of addressing SUD. The digital therapeutic application employs a single integrated reward track, which directly reinforces abstinence while modifying rewards based on additional activity completion. The single integrated reward track can be individualized on a per-user basis, with which CM can be performed remotely. An integrated reinforcement structure that supports sustained engagement and behavior change without diluting the primary goal of abstinence also is different from conventional CM approaches.

Aspects of the present disclosure are directed to systems and methods for providing activities and verification tests to facilitate abstinence associated with substance use in users. The system can include one or more processors coupled with memory. The one or more processors can be configured to maintain, for a profile associated with a user, a data structure comprising an activity field and a verification field. The one or more processors can be configured to determine, via one or more event handlers executing on an application, that activity data generated responsive to the user performing an activity via the application satisfies an activity condition. The one or more processors can be configured to update, responsive to determining that the activity data satisfies the activity condition, the data structure to include an activity value corresponding to the activity field. The one or more processors can be configured to receive verification data associated with the user in accordance with a verification test taken by the user. The one or more processors can update, responsive to determining that the verification data satisfies a verification condition, the data structure to include a verification value corresponding to the verification field. The one or more processors can execute an operation to update the profile using a token based on the activity value and the verification value.

The one or more processors can be further configured to generate the token as a function of at least one of: (i) a number of updates of the data structure to include at least one activity value and at least one verification value, (ii) a time duration over which a plurality of activity datasets and verification datasets is received, (iii) a frequency of the updates of the data structure to include at least one activity value and at least one verification value over a time duration, (iv) a percentage of at least one activity value and at least one verification value updated in the data structure, (v) a type of activity, (vi) a type of verification test, or (vii) any combination of the foregoing. The function can include at least one of a probabilistic function, a defined sequence, or a model.

In various implementations, the one or more processors can be further configured to determine, via the one or more event handlers executing on the application, that subsequent activity data generated responsive to the user performing a subsequent activity via the application satisfies a subsequent activity condition. The one or more processors can be configured to update, responsive to determining that the subsequent activity data satisfies the subsequent activity condition, the data structure to include a subsequent activity value corresponding to a subsequent activity field. The one or more processors can be configured to receive subsequent verification data associated with the user in accordance with a subsequent verification test taken by the user. The one or more processors can be configured to update, responsive to determining that the subsequent verification data satisfies a subsequent verification condition, the data structure to include a subsequent verification value corresponding to a subsequent verification field. The one or more processors can be configured to execute a subsequent operation to update the profile using a subsequent token based on the data structure.

The one or more processors can be further configured to generate the token as a function of at least one of: (i) a time elapsed since generation of the token, (ii) a number of tokens generated for the profile, (iii) a type of token, or (iv) a magnitude and/or probability of the token. The one or more processors can be further configured to generate a plurality of tokens comprising at least the token and the subsequent token using the function, responsive to executing a corresponding plurality of operations. The one or more processors can be further configured to generate the token as a plurality of weights corresponding to (i) a number of updates of the data structure to include at least one activity value and at least one verification value, (ii) a time duration over which a plurality of activity datasets and verification datasets is received, (iii) a frequency of the updates of the data structure to include at least one activity value and at least one verification value over a time duration, (iv) a percentage of at least one activity value and at least one verification value updated in the data structure, (v) a type of activity, (vi) a type of verification test, (vii) a time elapsed since generation of the token, (viii) a number of tokens generated for the profile, (ix) a type of token, (x) a magnitude and/or probability of the token, or (xi) any combination of the foregoing

The one or more processors can be further configured to generate a plurality of tokens using the plurality of weights, responsive to executing a corresponding plurality of operations. The one or more processors can be further configured to update at least one of the plurality of weights, responsive to generating at least one of the plurality of tokens. The one or more processors can be further configured to transmit, to a user device executing the application, an instruction to prompt the user to perform the activity via the application and monitor, via the one or more event handlers, for generation of the activity data responsive to performance of the activity by the user via the application. The activity can be selected from an activity related to cognitive behavioral therapy, an activity related to a psychoeducation lesson, an activity related to an assessment, an activity related to a training exercise, an activity related to a tool, an activity related to attendance, or an activity related to a condition, disease, disorder, or symptom.

The one or more processors can be further configured to determine that the activity data generated responsive to the user performing the activity satisfies the activity condition via a computing device external to the application and update, responsive to determining that the activity data satisfies the activity condition, the data structure to include the activity value corresponding to the activity field. In various implementations, the one or more processors are further configured to transmit, to a user device executing the application, an instruction to perform the verification test based on at least one of: (i) data associated with a sample from the user, (ii) a biomarker obtained from the user, (iii) a measurement from an instrumentation device, (iv) uploading digital information to the application, (v) a verification of the user's location; or (vi) data associated with a laboratory test provided by the user or the laboratory, and receive, from the user device, the verification data associated with the user taking the verification test via the application. The one or more processors can be further configured to receive, from a computing device external to the application, the verification data associated with the user taking the remote test via the application in accordance with the verification test.

In various implementations, the verification test is based on at least one of: (i) a lab test, (ii) data associated with a sample from the user, (iii) a biomarker obtained from the user, (iv) a measurement from an instrumentation device, (v) uploading digital information to the application, or (vi) a verification of the user's location. The verification test can be executed to generate the verification data, including a score to indicate at least one of (i) a level of substance in the user or (ii) an absence of substance in the user. The substance can be selected from marijuana, cocaine, alcohol, heroin, amphetamines, opioids, nicotine, benzodiazepines, barbiturates, and metabolites thereof. The one or more processors can be further configured to monitor, during a time duration subsequent to updating the data structure to include the activity value corresponding to the activity field, for receipt of the verification data. The one or more processors can be further configured to monitor, during a time duration subsequent to updating the data structure to include the verification value corresponding to the verification field, for receipt of the activity data.

The one or more processors can be further configured to refrain from updating the data structure to include the activity value corresponding to the activity field, responsive to determining that the activity data does not satisfy the activity condition. The one or more processors can be further configured to generate a score indicating a number of updates to the data structure to include the activity value and the verification value. The one or more processors can be further configured to provide a graphical user interface identifying a plurality of scores over a plurality of timepoints, each score of the plurality of scores indicating a respective number of updates to the data structure. The one or more processors can be further configured to set an eligibility field of the data structure to an eligibility value to enable updating of the activity field and the verification field. The one or more processors can be further configured to provide, responsive to setting the eligibility field to the eligibility value, an instruction via the application to prompt the user to perform the activity and the verification test. The one or more processors can be further configured to monitor, responsive to providing the instruction, for the activity data and the verification data. The one or more processors can be further configured to set the eligibility field to the eligibility value, responsive to at least one of: (i) a completion of a previous activity and/or verification test, (ii) a number of completed activities and/or verification tests, or (iii) a percentage of activities and/or verification tests completed.

In various implementations, the one or more processors are further configured to identify the previous activity and/or verification test for which the eligibility field is to be the eligibility value based on applying historical data to a model. The one or more processors can be further configured to generate a score to indicate the probability of enabling the updating of the activity field and the verification field and set the eligibility field of the data structure to the eligibility value, responsive to the score satisfying a threshold. The one or more processors can be further configured to determine a number of times to set the eligibility field of the data structure to the eligibility value, to enable updating of the activity field and the verification field. The token can be a reward to induce performance of the activity and the verification test for abstinence from a substance associated with substance abuse disorder in the user. The one or more processors can be configured to execute the operation by transferring the token to an account data structure associated with the user. The one or more processors can be configured to provide a schedule indicating a time of the activity and/or a time of the verification test. The one or more processors can be configured to provide a notification indicating the time of the activity and/or a time of the verification test. The user can be at risk of or diagnosed with substance abuse disorder, and wherein the user is taking an effective amount of a medication to address the substance abuse disorder in partial concurrence with at least one of the activity or the verification test. The medication can be selected from acamprosate, naltrexone, naloxone, disulfiram, gabapentin, methadone, baclofen, bupropion, klonopin, Remeron, a GLP-1 receptor agonist, a GIP receptor agonist, or any combination thereof.

### BRIEF DESCRIPTION OF THE DRAWINGS

The foregoing and other objects, aspects, features, and advantages of the disclosure will become more apparent and better understood by referring to the following description taken in conjunction with the accompanying drawings, in which:
FIG. 1 depicts a block diagram of a system for presenting activities and verification tests to address substance use in users in accordance with an illustrative embodiment.
FIG. 2 depicts a block diagram for a process to provide an instruction to a user to perform an activity, in accordance with an illustrative embodiment.
FIG. 3 depicts a block diagram for a process to provide an instruction to the user to perform a verification test, in accordance with an illustrative embodiment.
FIG. 4 depicts a block diagram for a process to generate a token for the user, in accordance with an illustrative embodiment.
FIG. 5 depicts a block diagram for a process to determine the eligibility of the user, in accordance with an illustrative embodiment.
FIGs. 6A-B depict screenshots of sets of example user interfaces for performing an activity and verification test, in accordance with an illustrative embodiment.
FIG. 7 depicts a flow diagram of a method for updating a data structure for the user to address substance use in accordance with an illustrative embodiment.
FIG. 8 is a block diagram of a server system and a client computer system in accordance with an illustrative embodiment.

### DETAILED DESCRIPTION

For purposes of reading the description of the various embodiments below, the following enumeration of the sections of the specification and their respective contents may be helpful:

Section A describes systems and methods for performing contingency management to induce abstinence for a substance use disorder (SUD) in a user.

Section B describes a network and computing environment that may be useful for practicing embodiments described herein.

### A. Systems and Methods for Performing Contingency Management to Induce Abstinence for Substance Use Disorder (SUD) in Users

Presented herein are systems and methods for managing data structures to maintain data from disparate sources for monitoring verification. A digital therapeutic application described herein enables digital delivery and processing of treatment, such as recovery activities and verification tests, to target SUDs. The digital therapeutic application can systematically reinforce target behaviors, such as abstinence, with rewards to promote positive changes in behavior. Provisions of rewards are contingent upon successful completion of verification tests, which verify that the user is abstinent, thus encouraging maintenance and repetition of the target behavior over time. The verification tests may be performed remotely, facilitating access and supporting the engagement of the user with the digital therapeutic application. For example, the user may provide images or videos in response to a verification test, and the test results may be interpreted and processed by a digital platform. To complement the completion of verification tests, the digital therapeutic application can also provide recovery activities to reinforce indicators of positive behavior change related to abstinence. The recovery activities may include, for example, cognitive activities or educational videos within the application.

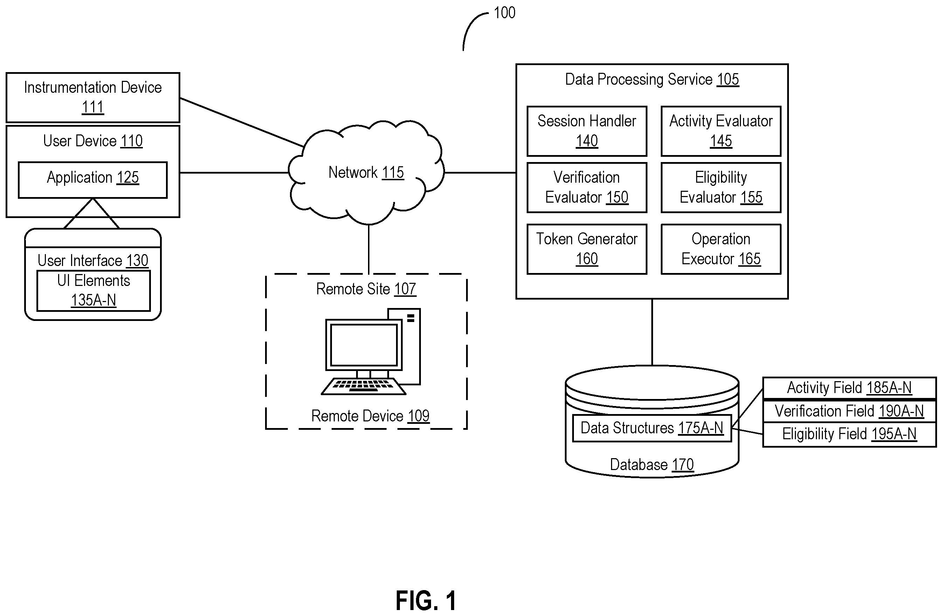

Referring now to FIG. 1, depicted is a block diagram of a system 100 for presenting interactive sessions to facilitate abstinence from substance use in users. In an overview, the system 100 may include at least one data processing service 105, at least one remote site 107, at least one user device 110, and at least one instrumentation device 111, communicatively coupled with one another via at least one network 115. The remote site 107 may include a remote device 109. The user device 110 may include at least one application 125. The application 125 may include or provide at least one user interface 130 with one or more user interface (UI) elements 135A-N (hereinafter generally referred to as UI elements 135). The data processing service 105 may include at least one session handler 140, activity evaluator 145, verification evaluator 150, eligibility evaluator 155, token generator 160, and operation executor 165, among others.

The data processing service 105 may include or have access to at least one database 170. The database 170 may store, maintain, or otherwise include one or more data structures 175 A-N (hereinafter generally referred to as data structures 175). The data structure 175 may be representative of a user profile, and include at least one or more activity fields 185 A-N (hereinafter generally referred to as activity fields 185), one or more verification fields 190 A-N (hereinafter generally referred to as verification fields 190), and one or more eligibility fields 195 A-N (hereinafter generally referred to as eligibility fields 195). The data structure 175 may be any type of data object maintained on the database 170 to keep track of the activity field 185, the verification field 190, or the eligibility field 195, among others. Collectively, the user device 110 and the data processing service 105 may be part of a computing system to provide the application 125.

In further detail, the data processing service 105 may (sometimes herein generally referred to as a service) be any computing device comprising one or more processors coupled with memory and software and capable of performing the various processes and activities described herein. The data processing service 105 may be in communication with the user device 110, the remote site 107, and the database 170 via the network 115. The data processing service 105 may be situated, located, or otherwise associated with at least one server group. The server group may correspond to a data center, a branch office, or a site at which one or more servers corresponding to the data processing service 105 is situated. The data processing service 105 may be situated, located, or otherwise associated with one or more of the user devices 110. Some components of the data processing service 105 may be located within the server group, and some may be located within the client device. For example, the data processing service 105 may operate or be situated on the user device 110, and the activity evaluator 145 may operate or be situated on the server group.

Within the data processing service 105, the session handler 140 may initiate sessions for a user by the application 125. The activity evaluator 145 may evaluate activity data received from the user device 110. The verification evaluator 150 may evaluate verification data received from the user device 110. The eligibility evaluator 155 may determine the eligibility of a user. The token generator 160 may generate a token based on the activity and verification data. The operation executor 165 may execute an operation to update a user profile based on the token.

The remote site 107 may be situated, located, or otherwise associated with at least one server group. The server group may correspond to a data center, a branch office, or a site at which one or more servers corresponding to the remote site 107 is situated. The remote site 107 may be situated, located, or otherwise associated with one or more of the user devices 110. The remote site 107 may be in communication with the user device 110 and the data processing service 105 via the network 115. For example, the remote site 107 may receive an image (including a video) from the user device 110 to process and verify abstinence of the user. The image may be an image of an eye, saliva, and/or hair of the user 210. The remote site 107 may process the image and transmit results of the processing to the data processing service 105. As another example, the remote site 107 may receive instruction from the data processing service 105 to provide a location of the user device 110 for the data processing service 105 to perform a verification test.

The remote site 107 may include the remote device 109 which may be any computing device comprising one or more processors coupled with memory and software and capable of performing the various processes and activities described herein. The remote device 109 can control, monitor, or interact with the user device 110 and the data processing service 105 via the network 115. The remote device 109 may also process information and/or instructions transmitted from at least one of the user device 110 or the data processing service 105. For example, the remote device 109 may process and generate results of an image provided by the user device 110. For example, the remote device 109 may detect an eye color of the user 210 based on the image, and generate the results based on the eye color. The remote site 107 may receive the image and provide the image to the remote device 109 to process and generate results. At least one of the remote site 107 or the remote device 109 can then transmit the results to, for example, the data processing service 105.

The user device 110 (sometimes herein referred to as an end-user computing device or client device) may be any computing device comprising one or more processors coupled with memory and software and capable of performing the various processes and activities described herein. The user device 110 may be in communication with the data processing service 105 and the database 170 via the network 115. The user device 110 may be a smartphone, other mobile phone, tablet computer, a wearable device (e.g., smart watch, eyeglasses), or laptop computer. The user device 110 may be used to access the application 125. In some embodiments, the application 125 may be downloaded and installed on the user device 110 (e.g., via a digital distribution platform). In some embodiments, the application 125 may be a web application with resources accessible via the network 115.

The application 125 executing on the user device 110 may be a digital therapeutics application and may provide sessions (sometimes herein referred to as a therapy session) to address substance use disorders (SUDs). The user of the application 125 may be diagnosed with, or at risk of, a SUD. For example, the user may be using a substance more frequently and in larger amounts, developing health problems such as respiratory issues, or having withdrawal symptoms, among others. The causes of developing SUDs can include genetic, behavioral, environmental, physiological, and psychological factors, among others. For example, individuals with a family history of SUDs may have an increased susceptibility to substances and are at a higher risk of developing an addiction. In another example, socioeconomic status, such as economic hardship and poverty, may contribute to the usage of substances as a coping mechanism.

Examples of SUDs include opioid use disorders, alcohol use disorders, narcotic use disorders, among others. SUDs can lead to physical health, mental health, social and economic, and other issues. Effects on physical health may include, for example, heart disease (e.g., hypertension, arrhythmia, or cardiomyopathy), reduced lung function, lung damage, brain damage (e.g., neurotoxicity), or immunosuppression, among others. The condition may impede or hinder relationships, such as facing social stigma, and may lead to economic losses such as unemployment.

The user may be at least partially concurrently receiving a treatment to address the condition, or side effects of the condition, at least partially concurrently with the interventions provided by the application 125. For example, the user may be receiving treatment for the SUD. The user may be receiving a treatment at least partially concurrently with any number of sessions, or any combination thereof. The treatment can include taking a medication. The medication may be at least orally administered, intravenously administered, or topically applied. For example, the medication may include acamprosate, naltrexone, naloxone, disulfiram, gabapentin, methadone, baclofen, bupropion, klonopin, Remeron, a GLP-1 receptor agonist, a GIP receptor agonist, or any combination thereof, among others. The application 125 may increase the efficacy of the medication that the user is taking to address the condition. The treatment can include cognitive behavioral therapy (CBT), contingency management, therapy, rehabilitation programs, or support groups, among others.

The application 125 may be used to provide verification tests and activities to the user to facilitate abstinence from substances. The activities may be targeted to mitigating substance use, such as activities relating to cognitive behavioral therapy, psychoeducation, or a training exercise. The verification tests may be targeted to verify continued abstinence of the user, such as a lab test, uploading digital information, or a location verification. By providing the user the digital therapeutics through the application 125, the adverse effects of SUDs can be addressed.

The application 125 can include, present, or otherwise provide a user interface 130 including the one or more UI elements 135 to a user of the user device 110 in accordance with a configuration on the application 125. The UI elements 135 may correspond to visual components of the user interface 130, such as a command button, a text box, a check box, a radio button, a menu item, and a slider, among others. In some embodiments, the application 125 may be a digital therapeutics application and may provide one or more sessions (sometimes referred to herein as a therapy session) via the user interface 130 for addressing substance use disorders in users.

The instrumentation device 111 (sometimes herein referred to as a wearable technology, wearable device, or device) may be a computing device capable of collecting measurements from the user 210. The instrumentation device 111 may be wearable technology worn by the user 210, such as a fitness tracker, watch, a pedometer, micro-needle device, or a heartbeat monitor, among others. The instrumentation device 111 may provide measurements to, for example, the remote device 109 or the data processing service 105 to determine to provide the user 210 with the verification test to perform. The instrumentation device 111 may be in communication with the data processing service 105, the remote site 107, the user device 110, and the database 170 via the network 115, among others.

The database 170 may store and maintain various resources and data associated with the data processing service 105 and the application 125. The database 170 may include a database management system (DBMS) to arrange and organize the data maintained thereon. The database 170 may be in communication with the data processing service 105 and the one or more user devices 110 via the network 115. While running various operations, the data processing service 105 and the application 125 may access the database 170 to retrieve identified data therefrom. The data processing service 105 and the application 125 may also write data onto the database 170 from running such operations.

Such operations may include the maintenance of the data structures 175. The database 170 may maintain the data structure 175. The data structure 175 may be included in a profile associated with a user. The data structure 175 can include information pertaining to a condition of a user (e.g., SUD), as described herein. For example, the data structure 175 may include information related to the severity of the condition, occurrences of the condition (such as occurrences of symptoms associated with the condition), medications or treatments the user takes for the condition, and/or a duration of the condition, among others. The data structure 175 can be updated responsive to a schedule, periodically (e.g., daily, weekly), responsive to a change in user information (e.g., input by the user via the user interface 130 or learned from the user device 110), or responsive to a clinician (e.g., a doctor or nurse) addressing the user's condition, among others. The data structure 175 may be used to track values corresponding to the completion of activities or tests by a user in furtherance of a unified reward track to carry out contingency management (CM).

The data structure 175 can store and maintain information related to a user of the application 125 through user device 110. Each data structure 175 may be associated with or correspond to a respective user of the application 125. The data structure 175 may contain or store information for each session performed by the user. The information for a session may include various parameters, actions, audio, images (including video), prompts, or selections or actions of previous sessions performed by the user and may initially be null. For example, the data structure 175 includes the activity field 185, the verification field 190, and the eligibility field 195. Each of the fields may store a value corresponding to, for example, completion of an activity, a verification test, or a determination of eligibility. The data structure 175 can enable streamlined communications to the user by presenting a session to the user, which, based on at least the data structure 175, is most likely to aid the user in addressing the SUD in the user. This directed approach can reduce the need for multiple communications with the user, thereby reducing bandwidth and increasing the benefit of the user-computer interaction.

In some embodiments, the data structure 175 may identify or include information on a treatment regimen undertaken by the user, such as a type of treatment (e.g., therapy, pharmaceutical, or psychotherapy), duration (e.g., days, weeks, or years), and frequency (e.g., daily, weekly, quarterly, or annually), among others. The data structure 175 may be stored and maintained in the database 170 using one or more files (e.g., extensible markup language (XML), comma-separated values (CSV) delimited text files, or a structured query language (SQL) file). The data structure 175 may be iteratively updated as the user provides responses, makes selections, and performs actions related to the session. The data structure 175 may also be updated based on values generated by the activity evaluator 145, the verification evaluator 150, the eligibility evaluator 155, and tokens generated by the token generator 160 by the operation executor 165.

On the database 170, each data structure 175 may include the activity field 185 corresponding to an activity value generated by the activity evaluator 145. The activity field 185 may be updated based on the activity value. The database 170 may include the verification field 190 corresponding to a verification value generated by the verification evaluator 150. The verification field 190 may be updated dependent on the completion and results of the verification test. The database 170 may include the eligibility field 195. The eligibility field 195 may correspond to an eligibility value generated by the eligibility evaluator 155. The eligibility field 195 may be set prior to the generation of the eligibility value. For example, a user may be determined to be eligible prior to a first session.

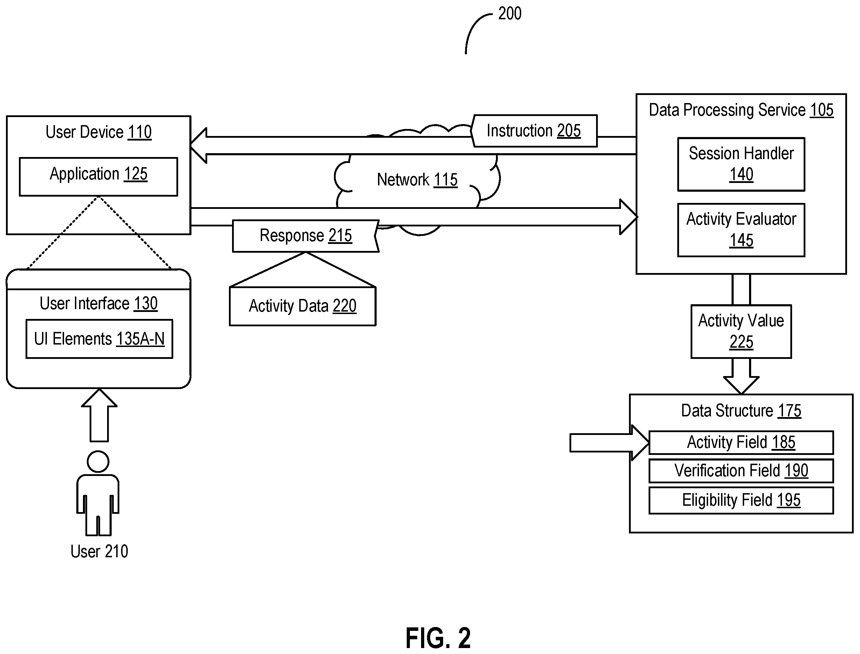

Referring now to FIG. 2, depicted is a block diagram for a process 200 to provide an instruction to a user 210 to perform an activity by the system 100. The process 200 may include or correspond to operations performed by the system 100 to receive and process data provided by the user 210. Under the process 200, the session handler 140 executing on the data processing service 105 may create, write, or otherwise generate one or more instructions 205 (herein generally referred to as instruction 205). The eligibility field 195 may have been set to indicate eligibility of the user 210 prior to the generation of the instruction 205. Eligibility of the user 210 may refer to eligibility of the user 210 to receive rewards based on verification of abstinence from substance use of the user 210. For example, the session handler 140 may refrain from generating the instruction 205 responsive to the eligibility field 195 indicating that the user 210 is not eligible (e.g., not abstinent).

The instruction 205 may include a prompt (e.g., message) to the user 210 to perform an activity via the application 125. The session handler 140 may select an activity to be included in the instruction 205. The session handler 140 may select the activity from an activity related to cognitive behavioral therapy (e.g., psychotherapy to change thought patterns), an activity related to a psychoeducation lesson (e.g., information about mental health conditions), an activity related to an assessment (e.g., test), an activity related to a training exercise (e.g., fitness), an activity related to a tool (e.g., within the app, related to mental health), an activity related to attendance (e.g., attending a group therapy session), or an activity related to a condition, disease, disorder, or symptom (e.g., learning about the disease).

In generating the instruction 205, the session handler 140 may identify and select the activity based at least on the data structure 175. The activities may be stored using one or more files on the database 170. For example, the activity may be a message with a prompt to go on a 10-minute walk. As another example, the activity may be an in-app (e.g., the application 125) or out-of-app activity, such as a cognitive activity or a psychoeducation lesson. The in-app activities may include therapeutic activities or recovery tools located within the application 125. For example, the application 125 can include educational videos regarding mental health, and the instruction 205 may request the user 210 to watch and take a quiz on one of the educational videos. The out-of-app activities may include verification of activity completion via location, activity monitoring, or electronic documentation. For example, verifying the location may ensure that the user 210 attended the group therapy session. The session handler 140 may select a different activity for a user with an alcohol use disorder than a user with an opioid use disorder. As another example, the session handler 140 may select the activity based on previous updates to the data structure 175, such as a time duration between setting the eligibility field 195 and generating the instruction 205.

With the generation, the session handler 140 may send, provide, or otherwise transmit the instruction 205 to the user device 110 (or the remote device 109 or the instrumentation device 111). The transmission of the instruction 205 may be in accordance with a schedule (e.g., at an interval of 1 day to 2 weeks). The instructions 205 may take various formats associated with the application 125. In some embodiments, the instructions 205 may be displayed, rendered, or otherwise presented via the user interface 130 of the application 125 to the user 210. In some embodiments, the instructions 205 may include a short message service (SMS) (e.g., text message) or multimedia message service (MMS) (e.g., audio message, video message).

In some embodiments, the instruction 205 may include a schedule indicating a time of the activity. For example, the instruction 205 may be provided to the user device 110 prior to the time at which the user 210 is instructed to perform the activity. The instruction 205 may indicate both a location and a time for which the user 210 is to perform the activity. The session handler 140 may also generate a notification for view on the user device 110 indicating the time of the activity for the user 210 to perform. The notification may appear via the UI elements 135 on the user device 110 for the user 210 to view. The instruction 205 may display the schedule indicating different, for examples, times of the day or days to perform the activity.

The application 125 on the user device 110 may retrieve, identify, or otherwise receive the instruction 205 from the data processing service 105. Upon receipt of the instruction 205, the application 125 on the user device 110 may parse the instruction 205 to identify the activity provided by the data processing service 105. In some embodiments, the application 125 can display, render, or otherwise present the user interface 130 using the instruction 205. The application 125 may then prompt or direct the user 210 to perform the activity as indicated by the instruction 205. Following transmission of the instruction 205, the session handler 140 may monitor for generation of activity data 220 as the user 210 is performing the activity via the application 125. For example, the application 125 may include one or more event handlers executing thereon. The event handlers may correspond to components on the user interface 130 of the application 125 that waits for an event to occur to monitor generation of the activity data 220. Upon detection of the performance in the activity, the application 125 may generate data associated with the activity as the user 210 is performing the activity. The application 125 may then fetch the activity data 220 once the user 210 completes the activity. Once the activity data 220 is generated, the application 125 may record a response 215 of the user 210, including the activity data 220. The response 215 may be indicative of a performance of the user 210 of the activity.

In some embodiments, the instrumentation device 111 (or the remote device 109) may retrieve, identify, or otherwise receive the instruction 205 directly from the data processing service 105 or indirectly via the application 125. Upon receipt of the instruction 205, the instrumentation device 111 may parse the instruction 205 to identify the activity provided by the data processing service 105. In some embodiments, the instrumentation device 111 can display, render, or otherwise present the user interface 130 using the instruction 205. The instrumentation device 111 may then prompt or direct the user 210 to perform the activity as indicated by the instruction 205. Following transmission of the instruction 205, the session handler 140 may monitor for generation of activity data 220 as the user 210 is performing the activity via the instrumentation device 111. For example, the instrumentation device 111 (or any application thereon) may include one or more event handlers executing thereon. The event handlers may correspond to components on the user interface 130 of the instrumentation device 111 that waits for an event to occur to monitor generation of the activity data 220. Upon detection of the performance in the activity, the instrumentation device 111 may generate data associated with the activity as the user 210 is performing the activity. The instrumentation device 111 may then fetch the activity data 220 once the user 210 completes the activity. Once the activity data 220 is generated, the instrumentation device 111 may record a response 215 of the user 210 including the activity data 220. The response 215 may be indicative of a performance of the user 210 of the activity.

The activity evaluator 145 may retrieve, identify, or otherwise receive the response 215 from the user device 110 (e.g., via the event handlers on the application 125) or the instrumentation device 111. With receipt, the activity evaluator 145 can determine whether the activity data 220 generated in response to the user 210 performing the activity via the application 125 satisfies an activity condition. The activity condition may be a precedent condition for completion of the activity for the activity field 185 of the data structure 175 to be updated. In some embodiments, the activity condition may be a threshold. For example, the activity condition can include a threshold of a percentage of the activity completed. The activity data 220 thus satisfies the activity condition responsive to being at or above the threshold. For example, responsive to the activity being a 10-minute walk, the activity data 220 representative of the 10-minute walk may satisfy the activity condition where the threshold is an 8-minute walk.

When the activity data 220 satisfies the activity condition, the activity evaluator 145 may generate at least one activity value 225. The activity value 225 may indicate a value to which to set to the activity field 185 to indicate completion of the activity indicated in the instruction 205. The activity value 225 may be a Boolean value, a numerical value, or an enumerated value, among others. The activity evaluator 145 may then update the data structure 175 with the activity value 225. For example, the activity value 225 corresponds to the activity field 185. The activity evaluator 145 can then update the activity field 185 with the activity value 225. Conversely, when the activity data 220 does not satisfy the activity condition, the activity evaluator 145 may refrain from generating the activity value 225 and from updating the data structure 175. For example, responsive to the activity being a 10-minute walk, the activity data 220 may indicate that the user 210 only walked for 7 minutes. In this case, activity evaluator 145 may determine that the activity data 220 does not satisfy the activity condition of the walk being at least 10 minutes. In some embodiments, the activity evaluator 145 may transmit or send a message or a notification to the user 210 indicating that the activity performed does not satisfy the activity condition.

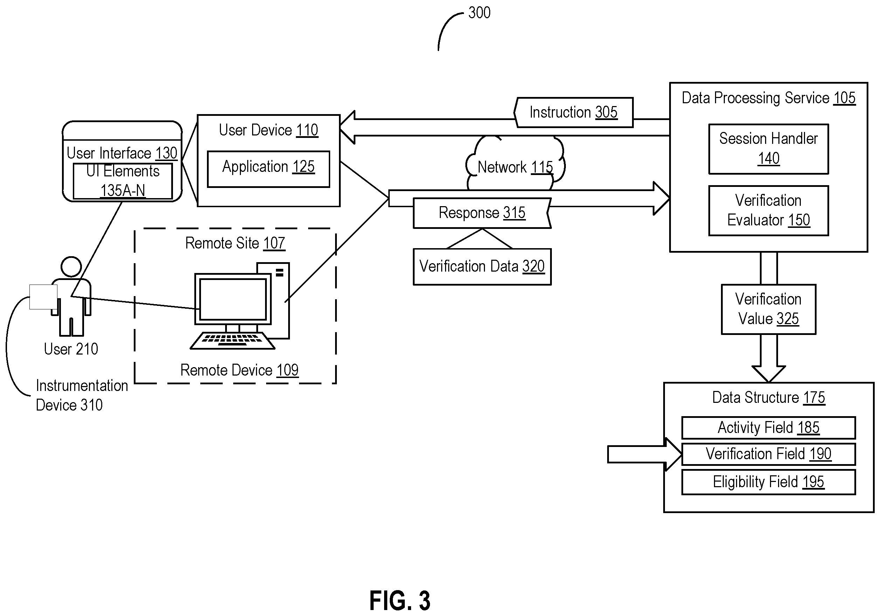

Referring now to FIG. 3, depicted is a block diagram for a process 300 to provide an instruction to a user 210 to take a verification test by the system 100. The process 300 may include or correspond to operations performed by the system 100 to receive and process data provided by the user 210. Under the process 300, the session handler 140 executing on the data processing service 105 may create, write, or otherwise generate one or more instructions 305 (herein generally referred to as instruction 305).

The instruction 305 may include a prompt or directions for the user 210 to perform a verification test to verify abstinence of the user 210 from substance use. The instruction 305 may indicate or identify the performance of the verification test. In some embodiments, the verification test may be performed via the user device 110 or the instrumentation device 111. In some embodiments, the verification test may be data associated with a sample from the user 210 (e.g., use of image data, audio data, or video data about the user 210). For instance, the verification test may be to verify abstinence by the user 210 from drug use, based on an image or video of an end-of-mouth swab that was pressed against the inner cheeks of the user 210. In some embodiments, the verification test may be based on a biomarker obtained from the user 210. The biomarker may include, for example, urine biomarkers (e.g., benzoylecgonine for cocaine, norcodeine for opioids, or tetrahydrocannabinol (THC) for marijuana), blood biomarkers (e.g., blood alcohol concentration, benzoylecgonine for cocaine, heroin metabolites, amphetamine levels), saliva biomarkers (e.g., THCs, heroin metabolites, amphetamine, or alcohol levels), breath biomarkers (e.g., ethanol), hair biomarkers (e.g., metabolites, THC, or benzodiazepines), among others.

In some embodiments, the verification test may be based on a measurement from the instrumentation device 111. The measurements may include various physiological measurements, such as irregular heartbeats indicative of tachycardia, slow respiration, sweat, cortisol levels, pupil dilations, among others. In some embodiments, the verification test may be uploading digital information to the application 125 (e.g., breathalyzer results, etc.). In some embodiments, the verification test may be a verification of the user 210's location. For instance, the verification test may be to identify the user's location and time based on at least one of the user device 110 or the instrumentation device 111. In some embodiments, the verification test may be data associated with a laboratory test provided by the user 210 or a laboratory (e.g., blood testing results).

In some embodiments, the verification test indicated in the instruction 305 may be performed at the remote site 107 or by the remote device 109 to verify the absence of the user 210 from substance use. In some embodiments, the verification test may be based on a lab test (e.g., blood, urine). For example, the verification test can be a virtual toxicology test, such as prompting the user 210 to take a photo of a saliva sample. In some embodiments, the verification test may be based on data associated with a sample from the user 210. For example, the verification test can be a remote verification test, and request the user 210 to take a photo of their eyes. In some embodiments, the verification test may be based on a biomarker obtained from the user 210. For example, the verification test can be an in-person point-of-care, lab-based, biomarker-based, or wearable device test. In some embodiments, the verification test may be based on a measurement from the instrumentation device 111 (e.g., blood pressure). In some embodiments, the verification test may be based on uploading digital information to the application 125 (e.g., daily exercise). In some embodiments, the verification test may be based on a verification of the user 210's location.

With the generation, the session handler 140 may send, provide, or otherwise transmit the instruction 305 to the user device 110, the remote device 109 or the instrumentation device 111. In some embodiments, the instruction 305 includes a schedule indicating a time of the verification test. For example, the instruction 305 may be provided prior to the time for the user 210 to take or input information regarding the verification test. In some embodiments, the data processing service 105 may schedule a verification test for the user 210 at the laboratory. The session handler 140 can generate a notification to the user device 110 notifying the user 210 to complete the verification test, in the case that the verification test is to be performed by the user 210 (e.g., opposed to verification tests performed by the instrumentation device 111 or the remote device 109). For example, the transmission for the instruction 305 may be based on a schedule depending on the substance use history of the user 210. For example, the instruction 305 may be provided to a user with a longer history of substance use more often than a user with a shorter history of substance use. In some embodiments, the instruction 305 includes a notification to the user 210 of the data processing service 105 receiving measurements from the instrumentation device 111 to perform a verification test. The session handler 140 may select the verification test based on the condition of the user 210. The session handler 140 may also select the verification test based on a number of updates to the verification field 190.

The application 125 on the user device 110 may retrieve, identify, or otherwise receive the instruction 305 from the data processing service 105 to perform the verification test via the user device 110. Upon receipt of the instruction 305, the application 125 may parse the instruction 305 to identify the verification test to be performed by the user 210. The application 125 may carry out, execute, or otherwise perform the verification test in accordance with the instruction 305. In some embodiments, the application 125 can display, render, or otherwise present the user interface 130 using the instruction 305. The application 125 can then prompt or direct the user 210 to perform the verification test responsive to receiving the instruction 305. For instance, in accordance with the instructions 305, the application 125 may prompt the user to take a mouth swab and then use the camera of the user device 110 to acquire an image (including a video) of the swab. Using the acquired image, the application 125 may apply computer vision (e.g., a trained machine learning model) to determine whether the user is abstinent or not abstinent from the substance.

In performing the verification test on the user 210, the application 125 may create, produce, or otherwise generate at least one response 315 including verification data 320. The verification data 320 can be indicative of the user 210 completing the verification test as well as the results of the verification test. For example, the verification data 320 may include confirmation of completion of a blood test, and include results of the blood test. In some embodiments, in generating the response 315, the application 125 may determine the verification data 320 to include a score indicative of a level of substance in the user 210. In some embodiments, in generating the response 315, the application 125 may determine the verification data 320 to include a score indicative of an absence of substance in the user 210. The substance can be selected from at least one of marijuana, cocaine, alcohol, heroin, amphetamines, opioids, nicotine, benzodiazepines, barbiturates, and metabolites thereof.

The instrumentation device 111 may retrieve, identify, or otherwise receive the instruction 305 from the data processing service 105 to perform the verification test via the instrumentation device 111. The instruction 305 may be received directly from the data processing service 105 or indirectly via the user device 110. The instrumentation device 111 may retrieve, identify, or otherwise receive the instruction 305 from the data processing service 105 to perform the verification test via the user device 110. Upon receipt of the instruction 305, the instrumentation device 111 may parse the instruction 305 to identify the verification test to be performed by the user 210. The instrumentation device 111 may carry out, execute, or otherwise perform the verification test in accordance with the instruction 305. For instance, in accordance with the instructions 305, the instrumentation device 111 may acquire physiological measurements from the user 210, such as heart rate or cortisol levels from a blood user. Using the acquired measurements, the instrumentation device 111 may determine whether the user is abstinent or not abstinent from the substance.

In performing the verification test on the user 210, the instrumentation device 111 may create, produce, or otherwise generate at least one response 315 including verification data 320. The verification data 320 can be indicative of the user 210 completing the verification test as well as the results of the verification test. For example, the verification data 320 may include confirmation of completion of a blood test, and include results of the blood test. In some embodiments, in generating the response 315, the instrumentation device 111 may determine the verification data 320 to include a score indicative of a level of substance in the user 210. For example, when the verification test is a biomarker-based test, the instrumentation device can generate or otherwise calculate the score indicating the level of substance in the user 210 based on a level of the biomarker detected in the user 210. In some embodiments, in generating the response 315, the instrumentation device 111 may determine the verification data 320 to include a score indicative of an absence of substance in the user 210.

The remote device 109 may retrieve, identify, or otherwise receive the instruction 305 from the data processing service 105 to perform the verification test on the user 210 at the remote site 107. The remote device 109 may retrieve, identify, or otherwise receive the instruction 305 from the data processing service 105 to perform the verification test via the remote device 109. The instruction 305 may be received directly from the data processing service 105 or indirectly via the user device 110. The remote device 109 may retrieve, identify, or otherwise receive the instruction 305 from the data processing service 105 to perform the verification test via the user device 110. Upon receipt of the instruction 305, the remote device 109 may parse the instruction 305 to identify the verification test to be performed by the user 210. The remote device 109 may carry out, execute, or otherwise perform the verification test in accordance with the instruction 305. For example, when the verification test is to verify the location of the user 210, the remote device 109 may determine whether the user 210 is at or not at a particular location as a proxy for determining whether the user 210 is continuing to be abstinent or not.

In performing the verification test on the user 210, the remote device 109 may create, produce, or otherwise generate at least one response 315 including verification data 320. The verification data 320 can be indicative of the user 210 completing the verification test as well as the results of the verification test. In some embodiments, in generating the response 315, the remote device 109 may determine the verification data 320 to include a score indicative of a level of substance in the user 210. In some embodiments, in generating the response 315, the remote device 109 may determine the verification data 320 to include a score indicative of an absence of substance in the user 210.

The verification evaluator 150 can retrieve, identify, or otherwise receive the response 315, including the verification data 320 from at least one of the application 125, the instrumentation device 111, or the remote device 109. Based on the verification data 320, the verification evaluator 150 may identify or determine whether the verification data 320 satisfies a verification condition (e.g., in a manner analogous to the activity condition). The verification condition may be a precedent condition for completion of the activity for the verification field 190 of the data structure 175 to be updated. In some embodiments, the verification condition may specify a threshold relating to the level of substance in the user 210 where the absence of substance in the user 210 is negligible or zero. The verification condition may depend on the SUD the user 210 has and/or the substance detected in the user 210. The verification condition may also depend on a type of the verification test. For example, a threshold for an ethanol biomarker test may be higher than a threshold for a morphine biomarker test.

When the verification data 320 satisfies the verification condition, the verification evaluator 150 can generate, calculate, or otherwise determine a verification value 325 corresponding to the verification field 190. The verification value 325 may be indicative of the level of substance or the absence of substance in the user 210. The verification evaluator 150 may generate the verification value 325 as a function of the level of substance detected in the user 210. The verification value 325 may be a numerical value, a Boolean value, or an enumerated value, among others. The verification evaluator 150 can then update the data structure 175 with the verification value 325. The verification value 325 may also be indicative of a period of time the user 210 has been abstinent from substance use. For example, the verification evaluator 150 may determine the verification value 325 as a function of time and the verification data 320.

When the verification data 320 does not satisfy the verification condition, the verification evaluator 150 refrains from generating the verification value 325 and updating the data structure 175. In this case, the session handler 140 may transmit a notification to the user 210 notifying the user 210 of the verification data 320 not satisfying the verification condition. The verification evaluator 150 may also receive a message from the user 210 indicating an excused absence. The verification evaluator 150 may receive the message responsive to determining that the verification data 320 does not satisfy the verification condition. Responsive to verifying the absence, the verification evaluator 150 may generate the verification value 325 based on the absence. In this case, the verification value 325 may be non-negative.

The above-described processes can be repeated any number of times. In some embodiments, the session handler 140 may generate subsequent instructions with prompts to perform an activity and/or to perform a verification test. Based on these instructions, the activity evaluator 145 can receive subsequent activity data, and determine that the subsequent activity data satisfies a subsequent activity condition in response to the user 210 performing a subsequent activity. The subsequent activity, the subsequent activity condition, and the subsequent activity data may be received and/or performed following the activity, the activity condition, and the activity data 220. The subsequent activity, the subsequent activity condition, and the subsequent activity data may be different than the activity, the activity condition, and the activity data 220. Responsive to determining that the subsequent activity data satisfies the subsequent activity condition, the activity evaluator 145 generates a subsequent activity value corresponding to a subsequent activity field. The activity evaluator 145 can then update the data structure 175 with the subsequent activity value. The activity evaluator 145 may generate the subsequent activity value following the generation of the activity value 225.

The verification evaluator 150 can also receive subsequent verification data associated with the user 210 in accordance with a subsequent verification test taken by the user 210. The subsequent verification test may be different from the verification test. When the subsequent verification data satisfies a subsequent verification condition, the verification evaluator 150 can update the data structure 175 with the subsequent verification value corresponding to a subsequent verification field. The subsequent verification field may be the verification field 190 updated with the verification value 325. The verification evaluator 150 may receive the subsequent verification data following receiving the verification data 320.

The verification evaluator 150 (or the activity evaluator 145) can also monitor, during a time duration subsequent to updating the data structure 175 with at least one of the activity value 225 or the verification value 325 for receipt of the verification data 320 or the activity data 220. The verification test may be provided to the user 210 within a predetermined time period following performance of the activity. The activity may be provided to the user 210 within a predetermined time period following performance of the verification test to maximize the effects of the activity. In some embodiments, the predetermined time period may be indicated in the schedule provided to the user 210 by at least one of the instruction 205 or the instruction 305. At least one of the instruction 205 or the instruction 305 may provide a time of the activity and a time of the verification test as well as a notification indicating the time.

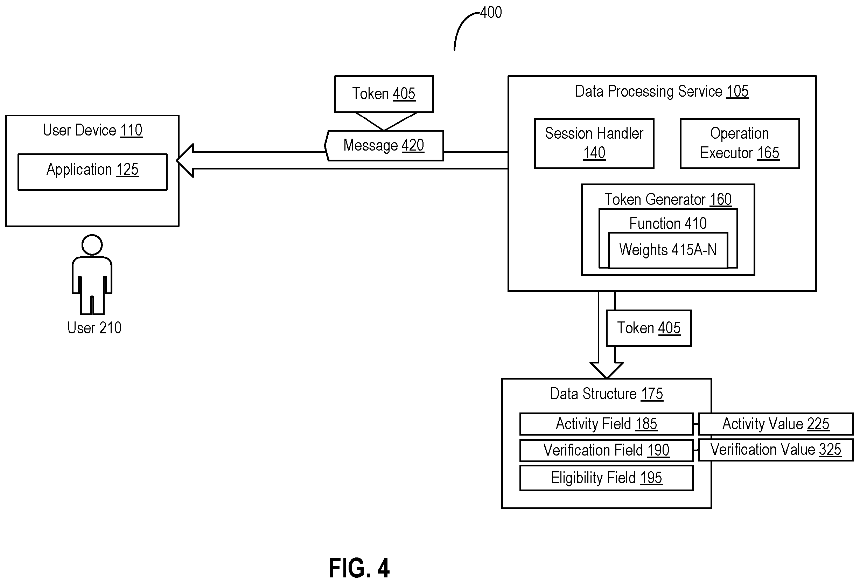

Referring now to FIG. 4, depicted is a block diagram for a process 400 to generate a token 405 responsive to the activity evaluator 145 and the verification evaluator 150 updating the data structure 175. The process 400 may include or correspond to operations performed by the system 100 to receive and process data provided by the user 210. Under the process 400, the token generator 160 may calculate, determine, or otherwise generate the token 405 based on at least the activity value 225 and the verification value 325. The generation of the token 405 may be in response to the setting of the activity value 225 to the activity field 185 and the verification value 325 to the verification field 190 in the data structure 175.

The token 405 may be a piece of data representing results of the performance of the activity and/or the verification test by the user 210. In some embodiments, the token 405 may be a reward (or positive reinforcement) to induce performance of the activity and the verification test for abstinence from a substance associated with the SUD of the user 210. The token 405 can thus be an incentive for the user 210 to both perform the activity and the verification test, but also for the activity data 220 and the verification data 320 generated from the completion of the activity and the verification test to satisfy the activity condition and the verification condition, respectively. The reward may be, for example, a monetary reward. The rewards may be intangible, such as certificates or badges reflecting progress made by the user 210. At the nervous system level, the token 405 may be provided to the user 210 to activate the brain's reward center (e.g., ventral striatum, prefrontal cortex, amygdala, anterior cingulate cortex, insular cortex, and hippocampus) to induce the user 210 to change their behavior and reduce substance abuse.

In some embodiments, the token 405 can represent various parameters, such as the probability of the token 405. The token 405 may be probabilistic. For example, a higher value (e.g., magnitude) of the token 405 may be generated in response to a longer period of time of verified abstinence as the probability of generating the higher value increases with time. As another example, the verification data 320 satisfying the verification condition may have a higher probability of generating a higher value token 405 compared to the activity data 220 satisfying the activity condition without the verification data 320 satisfying the verification condition.

With the updates of the data structure 175 with the activity value 225 and the verification value 325, the token generator 160 may generate token 405. The token generator 160 can include a function 410 to calculate, determine, or otherwise generate the token 405. The function 410 can include at least one of a probabilistic function, a defined sequence, or a model, among others. In general, the function 410 may include at least one input (e.g., at least a portion of the data structure 175 or an indication that the data structure 175 is updated) and at least one output corresponding to the token 405.

In some embodiments, the function 410 can be a probabilistic function, such as a random process or probability distribution (e.g., Bernoulli distribution, binomial distribution, geometric distribution, Poisson distribution, Zipf distribution, Gaussian distribution, exponential distribution, Gamma distribution, Chi-squared distribution, or Cauchy distribution). The probabilistic function may be used to generate a random value for the token 405. In some embodiments, the function 410 may be a defined sequence. The sequence may identify a sequence of values for the tokens to be provided for the user 210 over a set period (e.g., over the course of the activities and verification tests). The sequence may also include an association between an input (e.g., the number of updates) to a specified value of the token 405. In some embodiments, the defined sequence can include a formula or rule that dictates values of the sequence. In this case, the function 410 can generate the token 405 based on the rule by applying, for example, the number of updates to the data structure 175 to the rule.

In some embodiments, the function 410 can be a model. The function 410 can be a machine learning model, in accordance with an architecture. The architecture for the machine learning model can include, for example, a deep learning neural network (e.g., convolutional neural network architecture, a residual network, or a transformer-based architecture), a regression model (e.g., linear or logistic regression model), a random forest, a gradient boosting, a K-neighbors classifier and/or regressor, a support vector machine (SVM), a clustering algorithm (e.g., k-nearest neighbors), or a Naive Bayesian model, among others, and be supervised, unsupervised, or self-supervised. In general, the ML model may have at least one input and output. The input and output may be related via a set of weights according to the set of weights. The input may be data from the user, among others, while the output may include the value for the token 405.

The model for the function 410 can be trained using the training dataset. The training dataset may include a set of examples, including sample inputs (e.g., sample activity fields, verification fields, and eligibility fields of data structures) and sample outputs (e.g., values for tokens). To initialize, the values of the set of weights in the model for the function 410 to starting values (e.g., random, or defined values). To train, the sample input may be fed to the function 410 to generate an output token. With the output, the output token and the sample token may be compared. Based on the comparison, a loss metric may be determined in accordance with a loss function (e.g., a mean squared error, cross-entropy loss, hinge loss, or Huber loss). Using the loss metric, the one or more weights of the model may be updated. The updating of the weights may be in accordance with a back propagation and optimization function (sometimes referred to herein as an objective function) with one or more parameters (e.g., learning rate, momentum, weight decay, and number of iterations). The optimization function may define one or more parameters at which the weights of the model are to be updated. The optimization function may be in accordance with stochastic gradient descent, and may include, for example, an adaptive moment estimation (Adam), implicit update (ISGD), and adaptive gradient algorithm (AdaGrad), among others. The Model may be iteratively updated until convergence.

The token generator 160 can generate the token 405 by evaluating the function 410 of using any one or more of a number of updates of the data structure 175 to include at least one activity value 225 and at least one verification value 325 (e.g., a number of times the verification data 320 and the activity data 220 have satisfied the verification condition and the activity condition, respectively), a time duration over which a set of activity datasets and verification datasets is received, a frequency of the updates of the data structure 175 to include at least one activity value 225 and at least one verification value 325 over a time duration, a percentage of at least one activity value 225 and at least one verification value 325 updated in the data structure 175, a type of activity (e.g., training exercise, psychoeducation), or a type of verification test (e.g., lab, location), among others. The set of activity datasets and verification datasets may be a set of activity values 225 and verification values 325. The percentage of the activity value 225 and the verification value 325 may be based on a percentage of updates to the data structure 175 compared to a number of activities and/or verification tests performed by the user 210. For example, the token generator 160 can generate the token 405 based on the number of updates to the data structure 175 and a type of activity performed by the user 210.

In some embodiments, the token generator 160 can generate the token 405, dependent on previous generation of tokens. The previous generation of the tokens may be maintained on the data structure 175. In some embodiments, the token generator 160 can generate the token 405 as the function 410 of any one or more of the following: a time elapsed since generation of the token 405 (e.g., time between token generation), a number of tokens 405 generated for the profile (e.g., the data structure 175), a type of token 405 (e.g., based on type of verification test), or a magnitude and/or probability of the token 405 (e.g., value), among others. For example, the token generator 160 can use the previous generation of tokens as an input to the function 410 to generate the value for the next token 405 to assign to the data structure 175 for the user 210.

In some embodiments, the function 410 can also include a set of weights 415A-N (herein referred to as the set of weights 415). The token generator 160 can generate the token 405 according to and/or as the set of weights 415. The set of weights 415 can correspond to any one or more of the following: a number of updates of the data structure 175 to include at least one activity value 225 and at least one verification value 325; a time duration over which a set of activity datasets and verification datasets is received; a frequency of the updates of the data structure 175 to include at least one activity value 225 and at least one verification value 325 over a time duration; a percentage of at least one activity value 225 and at least one verification value 325 updated in the data structure 175; a type of activity; a type of remote verification test; a time elapsed since generation of the token 405;a number of tokens 405 generated for the profile (e.g., the data structure 175), a type of token 405; a magnitude and/or probability of the token 405, among others. Using the weights 415 of the function 410, the token generator 160 may evaluate the inputs (e.g., as listed herein) to generate the output token 405. With each evaluation, the token generator 160 may update at least one of the weights 415. The updating may be to set the value of the weights 415 to a random value (e.g., using a pseudo random number generator) to generate different values for the tokens 405.

With the generation of the token 405, the operation executor 165 can execute an operation to update or otherwise adjust the profile using the token 405 based on the activity value 225 and the verification value 325. The profile can be associated with the data structure 175. The data structure 175 of the profile can be updated to include the token 405 as well as the progress of the user 210 towards sustained abstinence from substance use. The operation may be to include, add, or otherwise insert the token 405 in the data structure 175. With the updating of the data structure 175, the operation executor 165 may store and maintain the data structure 175 on the database 170. In some embodiments, the operation executor 165 may carry out, perform, or otherwise execute the operation to transfer the token 405 to an account data structure associated with the user 210. The account data structure may be included or indicated by the profile. The account data structure can correspond, for example, to a bank account of the user 210.

In some embodiments, over the course of a time period, the token generator 160 can generate a set of tokens (e.g., including the token 405 and the subsequent token) using the function 410 responsive to the operation executor 165 executing a corresponding set of operations. For example, the token generator 160 can generate multiple tokens over a time period. The operation executor 165 can perform operations to update the profile based on each token generated by the token generator 160. In some embodiments, the token generator 160 can also generate the set of tokens based on the set of weights 415. In some embodiments, the token generator 160 generates at least one token 405 based on both the function 410 and the set of weights 415.

In conjunction with updating the data structure 175, the operation executor 165 may also generate at least one message 420 to include or indicate the token 405. The message 420 may include the token 405 to provide a presentation of the value of the token 405 via the application 125 to the user 210. With the generation, the operation executor 165 can send, transmit, or otherwise provide the message 420 to the user device 110 for presentation via the application 125. The message 420 may also include an indication regarding updates made to the profile and/or the account data structure of the user 210. The user 210 can thus view the token 405 as well as updates made to at least one of the profile or the account data structure by the operation executor 165. At the nervous system level, providing and presentation of the token 405 to the user can activate the brain's reward center (e.g., ventral striatumm, prefrontal cortex, amygdala, anterior cingulate cortex, insular cortex, and hippocampus) to help users change their behaviors and reduce substance abuse.

In some embodiments, the operation executor 165 can generate a score based on a number of updates to the data structure 175 with the activity value 225 and/or the verification value 325. The score may be indicative of a number of activities or verification tests performed by the user 210 that satisfied the activity condition and the verification condition, respectively. The score may be a function of the activity value 225, the verification value 325, and the number of updates to the data structure 175. Both the activity evaluator 145 and the operation executor 165 may track a number of updates to the data structure 175. The operation executor 165 can generate the score over a period of time.

For example, the operation executor 165 can generate a set of scores over a set of timepoints to indicate the progress of the user 210. The set of scores over the set of timepoints may represent continued abstinence of the user 210. The set of scores over the set of timepoints may be provided to the user 210 via a graphical user interface (e.g., the user interface 130) which can identify the set of scores over the set of timepoints. For example, the set of scores over the set of timepoints can be displayed as a graph on the user interface 130. Each score of the set of scores can indicate a respective number of updates to the data structure 175. For example, over the set of timepoints, the score may increase, reflecting an increasing number of updates to the data structure 175.

The above-mentioned processes may be repeated a number of times. Subsequent sessions (e.g., providing instructions to perform an activity or a verification test) may occur periodically. For example, the sessions may be provided 1 to 10 times weekly. The sessions may last for at least 5-15 weeks in total. During the time of the sessions, the user 210 may experience an escalating schedule of reinforcement. For example, a value of the token 405 may increase as the verification evaluator 150 generates more of the verification value 325. In some embodiments, the token generator 160 can also generate a subsequent token responsive to receiving the subsequent activity value and subsequent verification value. The operation executor 165 can thus execute a subsequent operation to update the profile using the subsequent token based on the data structure 175 (e.g., the subsequent activity value and verification value).

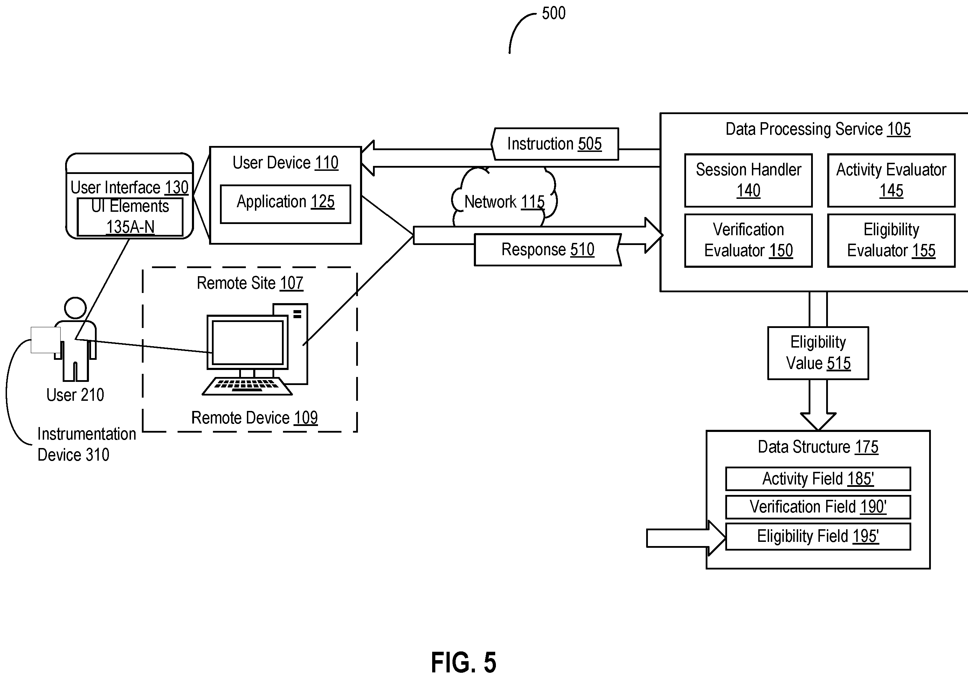

Referring now to FIG. 5, depicted is a block diagram for a process 500 to determine eligibility of the user 210. The process 500 may include or correspond to operations performed by the system 100 to receive and process data provided by the user 210. Under the process 500, the eligibility evaluator 155 may generate an instruction 505 to the user 210 to indicate the eligibility of the user 210. Prior to providing the instruction 205 or the instruction 305 to the user device 110, the eligibility evaluator 155 may set, update, or otherwise adjust the eligibility field 195' to an eligibility value 515 to enable updating of the activity field 185' and the verification field 190'. For example, to generate the token 405, the eligibility value 515 of the eligibility field 195' must be set. The eligibility value 515 may be a numerical or Boolean value.

Eligibility of the user 210 may depend on, for example, a number of activity completions. For example, the eligibility evaluator 155 may set the eligibility field 195' responsive to at least one of: a completion of a previous activity and/or verification test, a number of completed activities and/or verification tests, or a percentage of activities and/or verification tests completed (e.g., compared to a number of prompts to perform activities and verification tests), among others. The setting of the eligibility field 195' may enable the generation of the token 405 by the token generator 160. For example, responsive to the eligibility field 195 not being set or not satisfying a condition, the token generator 160 does not generate the token 405.

In some embodiments, the eligibility evaluator 155 can identify previous activity and/or verifications tests performed by the user 210 to set the eligibility field 195' based on applying historical data to a model (e.g., machine learning model). For example, based on the previous activity and/or verification tests, the eligibility evaluator 155 can set the eligibility field 195' to the eligibility value 515. As another example, the eligibility evaluator 155 can apply previous activity and verification values to the machine learning model to determine the eligibility value 515 of the user 210. The historical data may also include data from other users (e.g., not the user 210).

In some embodiments, the eligibility evaluator 155 can generate a score. The score may indicate the probability of enabling the updating of the activity field 185' and the verification field 190'. The eligibility evaluator 155 may generate the score prior to, concurrently with, or following the generation of the activity value 225 and the verification value 325. In some embodiments, the score may be based on the activity data 220 and the verification data 320 as well as the activity condition and the verification condition. The score may be used to determine whether the data structure 175 associated with the user is eligible to be assigned with a token. With the generation of the score, the eligibility evaluator 155 may then compare the score with a threshold.

When the score satisfies (e.g., greater than or equal to) the threshold, the eligibility evaluator 155 can set the eligibility field 195' of the data structure 175 to the eligibility value 515. The eligibility value 515 may be at least partially based on the score. The eligibility evaluator 155 setting the eligibility field 195' may also be dependent on a number of times the eligibility field 195 has been set to the eligibility value 515 which enables updating of the activity field 185' and the verification field 190'. The eligibility evaluator 155 may take into account a number of previous eligibilities, as well as a time period between each eligibility to determine whether to set the eligibility field 195 to the eligibility value 515. Conversely, when the score does not satisfy (e.g., less than) the threshold, the eligibility evaluator 155 can set the eligibility field 195' of the data structure 175 to the eligibility value 515.

In some embodiments, the eligibility evaluator 155 determining the eligibility value 515 may be performed in conjunction with or sequentially with the activity evaluator 145 and the verification evaluator 150 generating the activity value 225 and the verification value 325, respectively. For example, the eligibility evaluator 155 can set the eligibility field 195' responsive to updates to the activity field 185 and/or the verification field 190. The eligibility evaluator 155 may generate the eligibility value 515 for each update to at least the verification field 190'.

For example, the eligibility evaluator 155 setting the eligibility field 195' to the eligibility value 515 may be dependent on the verification data 320 satisfying the verification condition. Thus, the user 210 can only receive updates to their profile via the token 405 depending on the verification value 325. In this case, the activity value 225 can supplement the generation of the token 405 and may affect a value of the token 405, while the token generator 160 generating the token 405 is dependent on the verification value 325. Thus, abstinence is directly reinforced while recovery activity completion is indirectly reinforced. By targeting abstinence directly, it ensures that the value of the token 405 is not diluted by the completion of other target behaviors, but is rather associated with the highest priority behavior, abstinence.

With the setting of the eligibility field 195', the session handler 140 can transmit, generate, or otherwise provide the instruction 505 to the user 210 to prompt the user 210 to perform the activity and the verification test (e.g., as discussed in processes 200-400). Upon receipt of the instruction 505, the application 125, for example, can then monitor, via the event handlers, for the activity data 220 and the verification data 320. Once the activity evaluator 145 and the verification evaluator 150 have received the activity data (e.g., the activity data 220) and the verification data (e.g., the verification data 320) via a response 510, respectively, the activity evaluator 145 and the verification evaluator 150 can generate the activity value 225 and the verification value 325, respectively. The response 510 may also be provided to the eligibility evaluator 155.

The eligibility evaluator 155 can retrieve, identify, or otherwise receive the response 510 including the activity data 220 and the verification data 320. In some embodiments, the eligibility evaluator 155 retrieves, identifies, or otherwise receives the activity value 225 and the verification value 325 from the activity evaluator 145 and the verification evaluator 150, respectively. In some embodiments, the eligibility evaluator 155 may receive the verification data 320 or the verification value 325. Based on at least one of the verification data 320 or the verification value 325, the eligibility evaluator 155 generates the eligibility value 515. For example, the eligibility evaluator 155 may check the verification data 320 or the verification value 325 against an eligibility condition. The eligibility condition may be different than the verification condition. The eligibility evaluator 155 then determines whether the verification data 320 satisfies the eligibility condition. In some embodiments, the eligibility condition is the verification condition, and the eligibility evaluator 155 generates the eligibility value 515 based on the verification value 325. For example, the eligibility value 515 may be set responsive to the eligibility evaluator 155 receiving the verification value 325.

In some embodiments, responsive to the verification data satisfying the eligibility condition, the eligibility evaluator 155 generates the eligibility value 515. The eligibility evaluator 155 then updates or otherwise sets the eligibility field 195' with the eligibility value 515. In some embodiments, the eligibility evaluator 155 determines whether the activity data 220 and/or the verification data 320 satisfies the eligibility condition. In some embodiments, the eligibility evaluator 155 generates the eligibility value 515 based on the activity value 225 and/or the verification value 325. For example, the eligibility evaluator 155 may generate the eligibility value 515 based on the verification value 325 (e.g., the verification data 320 satisfying at least one of the verification condition or the eligibility condition), and adjust the eligibility value 515 based on the activity value 225. For example, completion of activities as indicated by the activity value 225 may affect the eligibility value 515. A higher number of activities completed may increase the eligibility value 515 compared to a lower number of activities completed, as indicated by the activity value 225. A higher eligibility value 515 may enable the user 210 to be eligible for a higher value of rewards and/or a higher token 405 value.

In this manner, by carrying out CM as described herein with the digital therapeutic application, the use of the data structure 175 for the single integrated reward track can eliminate redundant computations and storage, relative to approaches that would use separate and parallel tracks. From leveraging psychological and behavioral sciences, the digital therapeutic application may optimize reinforcement algorithms to provide a more targeted, individualized reinforcement to maximize the likelihood of clinical outcome (e.g., abstinence). At the same time, consumption of computing resources and network bandwidth may be reduced on the part of the data processing service 105 and the user device 110.

The data processing service 105 may compile both activity data 220 and verification data 320 to determine both eligibility and rewards to motivate the user 210 and enhance longevity and sustainability of abstinence. By leveraging both recovery activities and verification tests, the data processing service 105 can ensure continued, accurate assessments of abstinence while also providing activities to aid in the primary goal of abstinence. The data processing service 105 may combine behavioral management in digital therapeutics to create an accessible, comprehensive abstinence solution, thereby offering a more effective approach to addressing SUD conditions in the user 210.

Moreover, the data processing service 105 may support and maintain the data structure 175 to store values using multiple data formats aggregated over multiple devices, such as the remote device 109, the instrumentation device 111, or the user device 110, thereby improving interoperability capabilities of the data processing service 105. Since the data structure 175 does not rely on particular data formats of the respective devices, the data processing service 105 is able to interface with these devices and maintain data for remote verification of activities and tests, thereby saving consumption of memory in storing such data. The data structure 175 may allow for the conducting of the contingency management to track a user's completion of activities and verification tests for a unified reinforcement track, addressing technical challenges in conventional CM approaches. The data structure 175 can continuously and simultaneously update the activity field 185, the verification field 190, and the eligibility field 195 in real-time as data is received to accurately reflect progress, and timely provide reinforcement to the user 210.

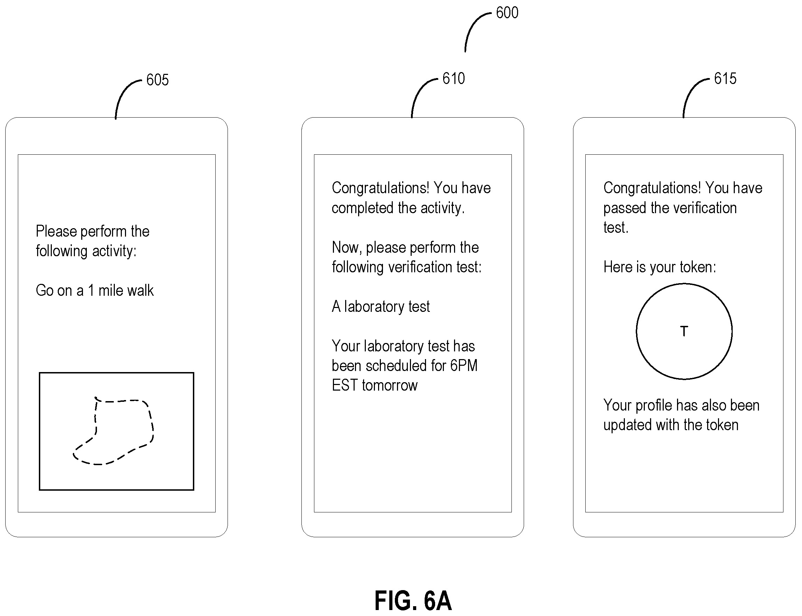

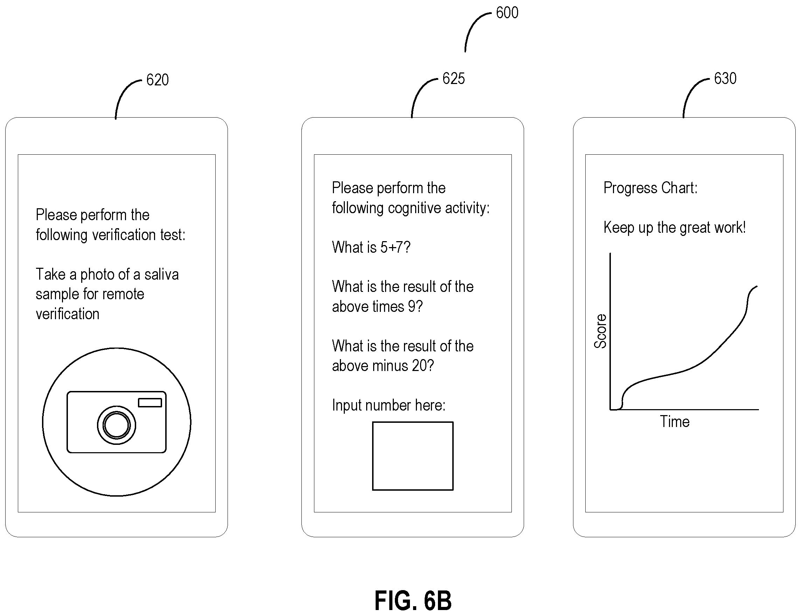

FIGS. 6A-B depict screenshots of a set 600 of user interfaces for performing activities and verification tests to generate a token, in accordance with an illustrative embodiment. The user interfaces in the set 600 may be part of the application 125, and presented through the user interface 130. The user interface 605 can provide a prompt to the user 210 to perform an activity. The activity may be related to a training exercise, such as walking. The user interface 610 can provide an indication of the user's completion of the activity, such as activity data received from the user satisfying an activity condition. The user interface 610 can also provide a prompt to the user 210 to perform a verification test. The user interface 615 can provide an indication of the verification data satisfying a verification condition, and include a message showing the token generated based on both the activity data and the verification data. The user interface 620 can provide a prompt to the user to complete a remote verification test. The remote verification test can be, for example, requesting an image (including a video) of the user's saliva. The remote verification test can also be images of the user's face, hair, or body, among others. The user interface 625 can prompt the user to complete a recovery activity via the user interface 625. For example, the user interface 625 can allow the user to complete a cognitive activity. The user interface 630 provides a display of a progress of the user over time. The user interface 630 can indicate the set of scores generated over a set of time points.

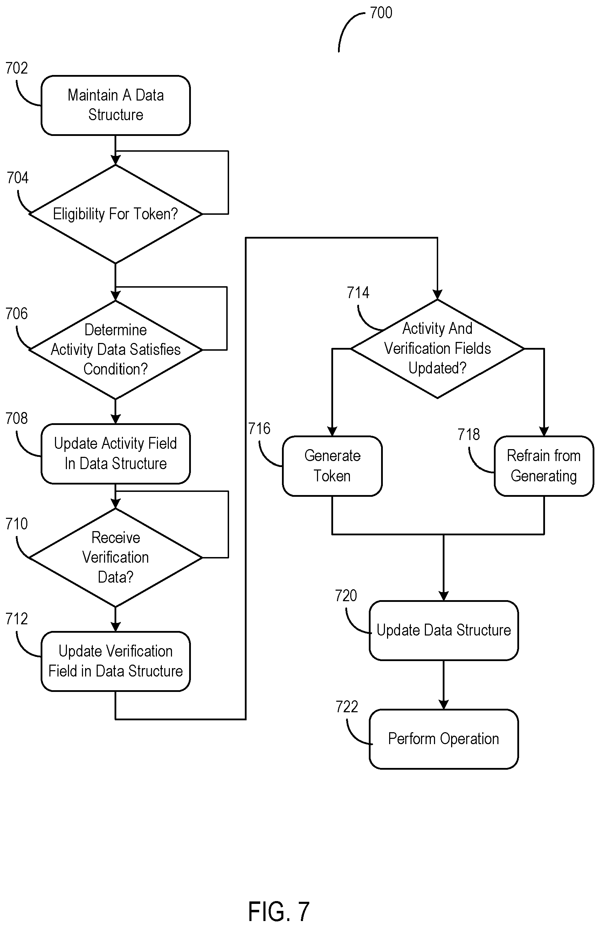

FIG. 7 depicts a flow diagram of a method 700 for providing activities and verification tests to users to address substance use disorders in accordance with an illustrative embodiment. The method 700 may be performed by any components of the system 100, such as the data processing service 105, the user device 110, or the user 210, among others. Under the method 700, a computing system can maintain a data structure (702). The data structure may be associated with a profile of a user. The computing system may determine whether the user is eligible for a token (704). The computing system can determine whether activity data received from the user satisfies a condition (706). Responsive to the activity data satisfying the condition, the computing system can update an activity field in the data structure (708). The computing system can update the activity field with an activity value generated based on the activity data. The computing system can determine whether verification data has been received (710). The verification data may be received responsive to providing an instruction to perform a verification test. Responsive to determining that verification data has been received, the computing system can update a verification field of the data structure (712). The verification field may be updated with a verification value generated based on the verification data.

The computing system can determine whether the activity and verification fields have been updated (714). Updating of the activity and verification fields can be dependent on the activity data and the verification data satisfying an activity condition and a verification condition. Responsive to determining that the activity and verification fields have been updated, the computing system can generate a token (716). Responsive to determining that the activity and verification fields have not been updated, the computing system can refrain from generating a token (718). The computing system can then update the data structure based on the token or a lack of the token (720). For example, the token or lack thereof may determine subsequent activities provided to the user. The computing system may then perform an operation (722). The operation may include updating the profile of the user associated with the data structure.

### B. Network and Computing Environment

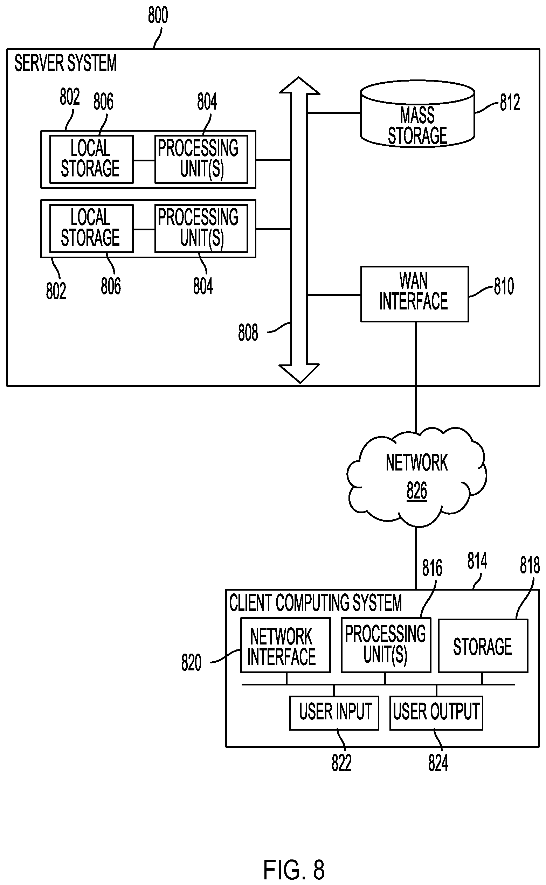

Various operations described herein can be implemented on computer systems. FIG. 8 shows a simplified block diagram of a representative server system 800, client computing system 814, and network 826 usable to implement certain embodiments of the present disclosure. In various embodiments, server system 800 or similar systems can implement services or servers described herein or portions thereof. Client computing system 814 or similar systems can implement clients described herein. The system 800 described herein can be similar to the server system 800. Server system 800 can have a modular design that incorporates a number of modules 802 (e.g., blades in a blade server embodiment); while two modules 802 are shown, any number can be provided. Each module 802 can include processing unit(s) 804 and local storage 806.

Processing unit(s) 804 can include a single processor, which can have one or more cores, or multiple processors. In some embodiments, processing unit(s) 804 can include a general-purpose primary processor as well as one or more special-purpose co-processors such as graphics processors, digital signal processors, or the like. In some embodiments, some or all processing unit(s) 804 can be implemented using customized circuits, such as application specific integrated circuits (ASICs) or field programmable gate arrays (FPGAs). In some embodiments, such integrated circuits execute instructions that are stored on the circuit itself. In other embodiments, processing unit(s) 804 can execute instructions stored in local storage 806. Any type of processors in any combination can be included in processing unit(s) 804.

Local storage 806 can include volatile storage media (e.g., DRAM, SRAM, SDRAM, or the like) or non-volatile storage media (e.g., magnetic or optical disk, flash memory, or the like). Storage media incorporated in local storage 806 can be fixed, removable, or upgradeable as desired. Local storage 806 can be physically or logically divided into various subunits such as a system memory, a read-only memory (ROM), and a permanent storage device. The system memory can be a read-and-write memory device or a volatile read-and-write memory, such as dynamic random-access memory. The system memory can store some or all of the instructions and data that processing unit(s) 804 need at runtime. The ROM can store static data and instructions that are needed by processing unit(s) 804. The permanent storage device can be a non-volatile read-and-write memory device that can store instructions and data even when module 802 is powered down. The term "storage medium" as used herein includes any medium in which data can be stored indefinitely (subject to overwriting, electrical disturbance, power loss, or the like) and does not include carrier waves and transitory electronic signals propagating wirelessly or over wired connections.

In some embodiments, local storage 806 can store one or more software programs to be executed by processing unit(s) 804, such as an operating system or programs implementing various server functions, such as functions of the system 800 or any other system described herein, or any other server(s) associated with system 800 or any other system described herein.

"Software" refers generally to sequences of instructions that, when executed by processing unit(s) 804, cause server system 800 (or portions thereof) to perform various operations, thus defining one or more specific machine embodiments that execute and perform the operations of the software programs. The instructions can be stored as firmware residing in read-only memory or program code stored in non-volatile storage media that can be read into volatile working memory for execution by processing unit(s) 804. Software can be implemented as a single program or a collection of separate programs or program modules that interact as desired. From local storage 806 (or non-local storage described below), processing unit(s) 804 can retrieve program instructions to execute and data to process in order to execute various operations described above.

In some server systems 800, multiple modules 802 can be interconnected via a bus or other interconnect 808, forming a local area network that supports communication between modules 802 and other components of server system 800. Interconnect 808 can be implemented using various technologies, including server racks, hubs, routers, etc.

A wide area network (WAN) interface 810 can provide data communication capability between the local area network (e.g., through the interconnect 808) and the network 826, such as the Internet. Other technologies can be used to communicatively couple the server system with the network 826, including wired (e.g., Ethernet, IEEE 802.3 standards) or wireless technologies (e.g., Wi-Fi, IEEE 802.11 standards).

In some embodiments, local storage 806 is intended to provide working memory for processing unit(s) 804, providing fast access to programs or data to be processed while reducing traffic on interconnect 808. Storage for larger quantities of data can be provided on the local area network by one or more mass storage subsystems 812 that can be connected to interconnect 808. Mass storage subsystem 812 can be based on magnetic, optical, semiconductor, or other data storage media. Direct attached storage, storage area networks, network-attached storage, and the like can be used. Any data stores or other collections of data described herein as being produced, consumed, or maintained by a service or server can be stored in mass storage subsystem 812. In some embodiments, additional data storage resources may be accessible via WAN interface 810 (potentially with increased latency).

Server system 800 can operate in response to requests received via WAN interface 810. For example, one of modules 802 can implement a supervisory function and assign discrete activities to other modules 802 in response to received requests. Work allocation techniques can be used. As requests are processed, results can be returned to the requester via WAN interface 810. Such operation can generally be automated. Further, in some embodiments, WAN interface 810 can connect multiple server systems 800 to each other, providing scalable systems capable of managing high volumes of activity. Other techniques for managing server systems and server farms (collections of server systems that cooperate) can be used, including dynamic resource allocation and reallocation.

Server system 800 can interact with various user-owned or user-operated devices via a wide-area network such as the Internet. An example of a user-operated device is shown in FIG. 8 as client computing system 814. Client computing system 814 can be implemented, for example, as a consumer device such as a smartphone, other mobile phone, tablet computer, wearable computing device (e.g., smart watch, eyeglasses), desktop computer, laptop computer, and so on.

For example, client computing system 814 can communicate via WAN interface 810. Client computing system 814 can include computer components such as processing unit(s) 816, storage device 818, network interface 820, user input device 822, and user output device 824. Client computing system 814 can be a computing device implemented in a variety of form factors, such as a desktop computer, laptop computer, tablet computer, smartphone, other mobile computing device, wearable computing device, or the like.

Processing unit 816 and storage device 818 can be similar to processing unit(s) 804 and local storage 806 described above. Suitable devices can be selected based on the demands to be placed on client computing system 814. For example, client computing system 814 can be implemented as a "thin" client with limited processing capability or as a high-powered computing device. Client computing system 814 can be provisioned with program code executable by processing unit(s) 816 to enable various interactions with server system 800.

Network interface 820 can provide a connection to the network 826, such as a wide area network (e.g., the Internet) to which WAN interface 810 of server system 800 is also connected. In various embodiments, network interface 820 can include a wired interface (e.g., Ethernet) or a wireless interface implementing various RF data communication standards such as Wi-Fi, Bluetooth, or cellular data network standards (e.g., 3G, 4G, LTE, etc.).

User input device 822 can include any device (or devices) via which a user can provide signals to client computing system 814; client computing system 814 can interpret the signals as indicative of particular user requests or information. In various embodiments, user input device 822 can include any or all of a keyboard, touch pad, touch screen, mouse or other pointing device, scroll wheel, click wheel, dial, button, switch, keypad, microphone, and so on.

User output device 824 can include any device via which client computing system 814 can provide information to a user. For example, user output device 824 can include display-to-display images generated by or delivered to client computing system 814. The display can incorporate various image generation technologies, e.g., a liquid crystal display (LCD), light-emitting diode (LED) display including organic light-emitting diodes (OLED), projection system, cathode ray tube (CRT), or the like, together with supporting electronics (e.g., digital-to-analog or analog-to-digital converters, signal processors, or the like). Some embodiments can include a device such as a touchscreen that function as both input and output device. In some embodiments, other user output devices 824 can be provided in addition to or instead of a display. Examples include indicator lights, speakers, tactile "display" devices, printers, and so on.

Some embodiments include electronic components, such as microprocessors, storage, and memory that store computer program instructions in a computer readable storage medium. Many of the features described in this specification can be implemented as processes that are specified as a set of program instructions encoded on a computer readable storage medium. When these program instructions are executed by one or more processing units, they cause the processing unit(s) to perform various operations indicated in the program instructions. Examples of program instructions or computer code include machine code, such as that produced by a compiler, and files including higher-level code that are executed by a computer, an electronic component, or a microprocessor using an interpreter. Through suitable programming, processing unit(s) 804 and 816 can provide various functionality for server system 800 and client computing system 814, including any of the functionality described herein as being performed by a server or client, or other functionality.

It will be appreciated that server system 800 and client computing system 814 are illustrative and that variations and modifications are possible. Computer systems used in connection with embodiments of the present disclosure can have other capabilities not specifically described here. Further, while server system 800 and client computing system 814 are described with reference to particular blocks, it is to be understood that these blocks are defined for convenience of description and are not intended to imply a particular physical arrangement of component parts. For instance, different blocks can be, but need not be, located in the same facility, in the same server rack, or on the same motherboard. Further, the blocks need not correspond to physically distinct components. Blocks can be configured to perform various operations, e.g., by programming a processor or providing appropriate control circuitry, and various blocks might or might not be reconfigurable depending on how the initial configuration is obtained. Embodiments of the present disclosure can be realized in a variety of apparatus including electronic devices implemented using any combination of circuitry and software.

While the disclosure has been described with respect to specific embodiments, one skilled in the art will recognize that numerous modifications are possible. Embodiments of the disclosure can be realized using a variety of computer systems and communication technologies, including but not limited to specific examples described herein. Embodiments of the present disclosure can be realized using any combination of dedicated components, programmable processors, or other programmable devices. The various processes described herein can be implemented on the same processor or different processors in any combination. Where components are described as being configured to perform certain operations, such configuration can be accomplished, e.g., by designing electronic circuits to perform the operation, by programming programmable electronic circuits (such as microprocessors) to perform the operation, or any combination thereof. Further, while the embodiments described above may make reference to specific hardware and software components, those skilled in the art will appreciate that different combinations of hardware or software components may also be used and that particular operations described as being implemented in hardware might also be implemented in software or vice versa.

Computer programs incorporating various features of the present disclosure may be encoded and stored on various computer readable storage media; suitable media include magnetic disk or tape, optical storage media such as compact disk (CD) or digital versatile disk (DVD), flash memory, and other non-transitory media. Computer readable media encoded with the program code may be packaged with a compatible electronic device, or the program code may be provided separately from electronic devices (e.g., via Internet download or as a separately packaged computer-readable storage medium).

Thus, although the disclosure has been described with respect to specific embodiments, it will be appreciated that the disclosure is intended to cover all modifications and equivalents within the scope of the following claims.

### EMBODIMENTS

1. A system, comprising:
   one or more processors coupled with memory, configured to:
   maintain, for a profile associated with a user, a data structure comprising an activity field and a verification field;
   determine, via one or more event handlers executing on an application, that activity data generated responsive to the user performing an activity via the application satisfies an activity condition;
   update, responsive to determining that the activity data satisfies the activity condition, the data structure to include an activity value corresponding to the activity field;
   receive verification data associated with the user in accordance with a verification test taken by the user;
   update, responsive to determining that the verification data satisfies a verification condition, the data structure to include a verification value corresponding to the verification field; and
   execute an operation to update the profile using a token based on the activity value and the verification value.
2. The system of clause 1, wherein the one or more processors are further configured to generate the token as a function of at least one of: (i) a number of updates of the data structure to include at least one activity value and at least one verification value, (ii) a time duration over which a set of activity datasets and verification datasets is received, (iii) a frequency of the updates of the data structure to include the at least one activity value and the at least one verification value over a time duration, (iv) a percentage of the at least one activity value and the at least one verification value updated in the data structure, (v) a type of activity, (vi) a type of verification test, or (vii) any combination of the foregoing.
3. The system of clause 2, wherein the function comprises at least one of a probabilistic function, a defined sequence, or a model.
4. The system of any preceding clause, wherein the one or more processors are further configured to:
   determine, via the one or more event handlers executing on the application, that subsequent activity data generated responsive to the user performing a subsequent activity via the application satisfies a subsequent activity condition;
   update, responsive to determining that the subsequent activity data satisfies the subsequent activity condition, the data structure to include a subsequent activity value corresponding to a subsequent activity field;
   receive subsequent verification data associated with the user in accordance with a subsequent verification test taken by the user;
   update, responsive to determining that the subsequent verification data satisfies a subsequent verification condition, the data structure to include a subsequent verification value corresponding to a subsequent verification field; and
   execute a subsequent operation to update the profile using a subsequent token based on the data structure.
5. The system of any preceding clause, wherein the one or more processors are further configured to generate the token as a function of at least one of: (i) a time elapsed since generation of the token, (ii) a number of tokens generated for the profile, (iii) a type of token, or (iv) a magnitude and/or probability of the token.
6. The system of clause 5, wherein the one or more processors are further configured to generate a set of tokens comprising at least the token and the subsequent token using the function, responsive to executing a corresponding set of operations.
7. The system of any preceding clause, wherein the one or more processors are further configured to generate the token as a set of weights corresponding to (i) a number of updates of the data structure to include at least one activity value and at least one verification value, (ii) a time duration over which a set of activity datasets and verification datasets is received, (iii) a frequency of the updates of the data structure to include the at least one activity value and the at least one verification value over a time duration, (iv) a percentage of the at least one activity value and the at least one verification value updated in the data structure, (v) a type of activity, (vi) a type of verification test, (vii) a time elapsed since generation of the token, (viii) a number of tokens generated for the profile, (ix) a type of token, (x) a magnitude and/or probability of the token, or (xi) any combination of the foregoing.
8. The system of clause 7, wherein the one or more processors are further configured to generate a set of tokens using the set of weights, responsive to executing a corresponding set of operations.
9. The system of clause 8, wherein the one or more processors are further configured to update at least one of the set of weights, responsive to generating at least one of the set of tokens.
10. The system of any preceding clause, wherein the one or more processors are further configured to:
   transmit, to a user device executing the application, an instruction to prompt the user to perform the activity via the application; and
   monitor, via the one or more event handlers, for generation of the activity data responsive to performance of the activity by the user via the application.
11. The system of clause 10, wherein the activity is selected from an activity related to cognitive behavioral therapy, an activity related to a psychoeducation lesson, an activity related to an assessment, an activity related to a training exercise, an activity related to a tool, an activity related to attendance, or an activity related to a condition, disease, disorder, or symptom.
12. The system of any preceding clause, wherein the one or more processors are further configured to:
   determine that the activity data generated responsive to the user performing the activity satisfies the activity condition via a computing device external to the application; and
   update, responsive to determining that the activity data satisfies the activity condition, the data structure to include the activity value corresponding to the activity field.
13. The system of any preceding clause, wherein the one or more processors are further configured to
   transmit, to a user device executing the application, an instruction to perform the verification test based on at least one of: (i) data associated with a sample from the user, (ii) a biomarker obtained from the user, (iii) a measurement from an instrumentation device, (iv) uploading digital information to the application, (v) a verification of the user's location; or (vi) data associated with a laboratory test provided by the user or a laboratory; and
   receive, from the user device, the verification data associated with the user taking the verification test via the application.
14. The system of any preceding clause, wherein the one or more processors are further configured to receive, from a computing device external to the application, the verification data associated with the user taking the remote test via the application in accordance with the verification test.
15. The system of clause 14, wherein the verification test is based on at least one of: (i) a lab test, (ii) data associated with a sample from the user, (iii) a biomarker obtained from the user, (iv) a measurement from an instrumentation device, (v) uploading digital information to the application, or (vi) a verification of the user's location.
16. The system of any preceding clause, wherein the verification test is executed to generate the verification data including a score to indicate at least one of (i) a level of substance in the user or (ii) an absence of substance in the user.
17. The system of clause 16, wherein the substance is selected from marijuana, cocaine, alcohol, heroine, amphetamines, opioids, nicotine, benzodiazepines, barbiturates, and metabolites thereof.
18. The system of any preceding clause, wherein the one or more processors are further configured to monitor, during a time duration subsequent to updating the data structure to include the activity value corresponding to the activity field, for receipt of the verification data.
19. The system of any preceding clause, wherein the one or more processors are further configured to monitor, during a time duration subsequent to updating the data structure to include the verification value corresponding to the verification field, for receipt of the activity data.
20. The system of any preceding clause, wherein the one or more processors are further configured to refrain from updating the data structure to include the activity value corresponding to the activity field, responsive to determining that the activity data does not satisfy the activity condition.
21. The system of any preceding clause, wherein the one or more processors are further configured to refrain from updating the data structure to include the verification value corresponding to the verification field, responsive to the verification data indicating a level of substance in the user.
22. The system of any preceding clause, wherein the one or more processors are further configured to generate a score indicating a number of updates to the data structure to include the activity value and the verification value.
23. The system of clause 22, wherein the one or more processors are further configured to provide a graphical user interface identifying a set of scores over a set of timepoints, each score of the set of scores indicating a respective number of updates to the data structure.
24. The system of any preceding clause, wherein the one or more processors are further configured to:
   set an eligibility field of the data structure to an eligibility value to enable updating of the activity field and the verification field;
   provide, responsive to setting the eligibility field to the eligibility value, an instruction via the application to prompt the user to perform the activity and the verification test, and
   monitor, responsive to providing the instruction, for the activity data and the verification data.
25. The system of clause 24, wherein the one or more processors are further configured to set the eligibility field to the eligibility value, responsive to at least one of: (i) a completion of a previous activity and/or verification test, (ii) a number of completed activities and/or verification tests, or (iii) a percentage of activities and/or verification tests completed.
26. The system of clause 24 or clause 25, wherein the one or more processors are further configured to identify the previous activity and/or verification test for which the eligibility field is to be to the eligibility value based on applying historical data to a model.
27. The system of any of clauses 24 to 26, wherein the one or more processors are further configured to:
   generate a score to indicate probability of enabling the updating of the activity field and the verification field; and
   set the eligibility field of the data structure to the eligibility value, responsive to the score satisfying a threshold.
28. The system of any of clauses 24 to 27, wherein the one or more processors are further configured to determine a number of times to set the eligibility field of the data structure to the eligibility value, to enable updating of the activity field and the verification field.
29. The system of any preceding clause, wherein the token is a reward to induce performance of the activity and the verification test for abstinence from a substance associated with substance abuse disorder in the user.
30. The system of any preceding clause, wherein the one or more processors are configured to execute the operation by transferring the token to an account data structure associated with the user.
31. The system of any preceding clause, wherein the one or more processors are configured to provide a schedule indicating a time of the activity and/or a time of the verification test.
32. The system of clause 31, wherein the one or more processors are configured to provide a notification indicating the time of the activity and/or a time of the verification test.
33. The system of any preceding clause, wherein the user is at risk of or diagnosed with substance abuse disorder, and wherein the user is taking an effective amount of a medication to address the substance abuse disorder in partial concurrence with at least one of the activity or the verification test.
34. The system of clause 33, wherein the medication is selected from acamprosate, naltrexone, naloxone, disulfiram, gabapentin, methadone, baclofen, bupropion, klonopin, Remeron, a GLP-1 receptor agonist, a GIP receptor agonist, or any combination thereof.
35. A method, comprising:
   maintaining, by one or more processors, for a profile associated with a user, a data structure comprising an activity field and a verification field;
   determining, by the one or more processors, via one or more event handlers executing on an application, that activity data generated responsive to the user performing an activity via the application satisfies an activity condition;
   updating, by the one or more processors, responsive to determining that the activity data satisfies the activity condition, the data structure to include an activity value corresponding to the activity field;
   receiving, by the one or more processors, verification data associated with the user in accordance with a verification test taken by the user;
   updating, by the one or more processors, responsive to determining that the verification data satisfies a verification condition, the data structure to include a verification value corresponding to the verification field; and
   executing, by the one or more processors, an operation to update the profile using a token based on the activity value and the verification value.
36. The method of clause 35, further comprising generating, by the one or more processors, the token as a function of at least one of: (i) a number of updates of the data structure to include at least one activity value and at least one verification value, (ii) a time duration over which a set of activity datasets and verification datasets is received, (iii) a frequency of the updates of the data structure to include the at least one activity value and the at least one verification value over a time duration, (iv) a percentage of the at least one activity value and the at least one verification value updated in the data structure, (v) a type of activity, (vi) a type of verification test, or (vii) any combination of the foregoing.
37. The method of clause 36, wherein the function comprises at least one of a probabilistic function, a defined sequence, or a model.
38. The method of any of clauses 35 to 37, further comprising:
   determining, by the one or more processors, via the one or more event handlers executing on the application, that subsequent activity data generated responsive to the user performing a subsequent activity via the application satisfies a subsequent activity condition;
   updating, by the one or more processors, responsive to determining that the subsequent activity data satisfies the subsequent activity condition, the data structure to include a subsequent activity value corresponding to a subsequent activity field;
   receiving, by the one or more processors, subsequent verification data associated with the user in accordance with a subsequent verification test taken by the user;
   updating, by the one or more processors, responsive to determining that the subsequent verification data satisfies a subsequent verification condition, the data structure to include a subsequent verification value corresponding to a subsequent verification field; and
   executing, by the one or more processors, a subsequent operation to update the profile using a subsequent token based on the data structure.
39. The method of any of clauses 35 to 38, further comprising the method further includes generating, by the one or more processors, the token as a function of at least one of: (i) a time elapsed since generation of the token, (ii) a number of tokens generated for the profile, (iii) a type of token, or (iv) a magnitude and/or probability of the token.
40. The method of clause 39, further comprising generating, by the one or more processors, a set of tokens comprising at least the token and the subsequent token using the function, responsive to executing a corresponding set of operations.
41. The method of any of clauses 35 to 40, further comprising generating, by the one or more processors, the token as a set of weights corresponding to (i) a number of updates of the data structure to include at least one activity value and at least one verification value, (ii) a time duration over which a set of activity datasets and verification datasets is received, (iii) a frequency of the updates of the data structure to include the at least one activity value and the at least one verification value over a time duration, (iv) a percentage of the at least one activity value and the at least one verification value updated in the data structure, (v) a type of activity, (vi) a type of verification test, (vii) a time elapsed since generation of the token, (viii) a number of tokens generated for the profile, (ix) a type of token, (x) a magnitude and/or probability of the token, or (xi) any combination of the foregoing.
42. The method of clause 41, further comprising generating, by the one or more processors, a set of tokens using the set of weights, responsive to executing a corresponding set of operations.
43. The method of clause 42, wherein the method further comprises updating, by the one or more processors, at least one of the set of weights, responsive to generating at least one of the set of tokens.
44. The method of any of clauses 35 to 43, further comprising:
   transmitting, by the one or more processors, to a user device executing the application, an instruction to prompt the user to perform the activity via the application; and
   monitoring, by the one or more processors, via the one or more event handlers, for generation of the activity data responsive to performance of the activity by the user via the application.
45. The method of clause 44, wherein the activity is selected from an activity related to cognitive behavioral therapy, an activity related to a psychoeducation lesson, an activity related to an assessment, an activity related to a training exercise, an activity related to a tool, an activity related to attendance, or an activity related to a condition, disease, disorder, or symptom.
46. The method of clause 45, further comprising:
   determining, by the one or more processors, that the activity data generated responsive to the user performing the activity satisfies the activity condition via a computing device external to the application; and
   updating, by the one or more processors, responsive to determining that the activity data satisfies the activity condition, the data structure to include the activity value corresponding to the activity field.
47. The method of any of clauses 35 to 46, further comprising:
   transmitting, by the one or more processors, to a user device executing the application, an instruction to perform the verification test based on at least one of: (i) data associated with a sample from the user, (ii) a biomarker obtained from the user, (iii) a measurement from an instrumentation device, (iv) uploading digital information to the application, (v) a verification of the user's location; or (vi) data associated with a laboratory test provided by the user or a laboratory; and
   receiving, by the one or more processors, from the user device, the verification data associated with the user taking the verification test via the application.
48. The method of any of clauses 35 to 47, further comprising receiving, by the one or more processors, from a computing device external to the application, the verification data associated with the user taking the remote test via the application in accordance with the verification test.
49. The method of clause 48, wherein the verification test is based on at least one of: (i) a lab test, (ii) data associated with a sample from the user, (iii) a biomarker obtained from the user, (iv) a measurement from an instrumentation device, (v) uploading digital information to the application, or (vi) a verification of the user's location.
50. The method of any of clauses 35 to 49, wherein the verification test is executed to generate the verification data including a score to indicate at least one of (i) a level of substance in the user or (ii) an absence of substance in the user.
51. The method of clause 50, wherein the substance is selected from marijuana, cocaine, alcohol, heroine, amphetamines, opioids, nicotine, benzodiazepines, barbiturates, and metabolites thereof.
52. The method of any of clauses 35 to 51, further comprising monitoring, by the one or more processors, during a time duration subsequent to updating the data structure to include the activity value corresponding to the activity field, for receipt of the verification data.
53. The method of any of clauses 35 to 52, further comprising monitoring, by the one or more processors, during a time duration subsequent to updating the data structure to include the verification value corresponding to the verification field, for receipt of the activity data.
54. The method of any of clauses 35 to 53, further comprising refraining, by the one or more processors, from updating the data structure to include the activity value corresponding to the activity field, responsive to determining that the activity data does not satisfy the activity condition.
55. The method of any of clauses 35 to 54, further comprising refraining, by the one or more processors, from updating the data structure to include the verification value corresponding to the verification field, responsive to the verification data indicating a level of substance in the user.
56. The method of any of clauses 35 to 55, further comprising generating, by the one or more processors, a score indicating a number of updates to the data structure to include the activity value and the verification value.
57. The method of clause 56, further comprising providing, by the one or more processors, a graphical user interface identifying a set of scores over a set of timepoints, each score of the set of scores indicating a respective number of updates to the data structure.
58. The method of any of clauses 35 to 57, further comprising:
   setting, by the one or more processors, an eligibility field of the data structure to an eligibility value to enable updating of the activity field and the verification field;
   providing, by the one or more processors, responsive to setting the eligibility field to the eligibility value, an instruction via the application to prompt the user to perform the activity and the verification test, and
   monitoring, by the one or more processors, responsive to providing the instruction, for the activity data and the verification data.
59. The method of clause 58, further comprising setting, by the one or more processors, the eligibility field to the eligibility value, responsive to at least one of: (i) a completion of a previous activity and/or verification test, (ii) a number of completed activities and/or verification tests, or (iii) a percentage of activities and/or verification tests completed.
60. The method of clause 58 or clause 59, further comprising identifying, by the one or more processors, the previous activity and/or verification test for which the eligibility field is to be to the eligibility value based on applying historical data to a model.
61. The method of any of clauses 58 to 60, further comprising:
   generating, by the one or more processors, a score to indicate probability of enabling the updating of the activity field and the verification field; and
   setting, by the one or more processors, the eligibility field of the data structure to the eligibility value, responsive to the score satisfying a threshold.
62. The method of any of clauses 58 to 61, further comprising determining, by the one or more processors, a number of times to set the eligibility field of the data structure to the eligibility value, to enable updating of the activity field and the verification field.
63. The method of any of clauses 35 to 62, wherein the token is a reward to induce performance of the activity and the verification test for abstinence from a substance associated with substance abuse disorder in the user.
64. The method of any of clauses 35 to 63, further comprising executing, by the one or more processors, the operation by transferring the token to an account data structure associated with the user.
65. The method of any of clauses 35 to 64, further comprising providing, by the one or more processors, a schedule indicating a time of the activity and/or a time of the verification test.
66. The method of clause 65, further comprising providing, by the one or more processors, a notification indicating the time of the activity and/or a time of the verification test.
67. The method of any of clauses 35 to 66, wherein the user is at risk of or diagnosed with substance abuse disorder, and wherein the user is taking an effective amount of a medication to address the substance abuse disorder in partial concurrence with at least one of the activity or the verification test.
68. The method of clause 67, wherein the medication is selected from acamprosate, naltrexone, naloxone, disulfiram, gabapentin, methadone, baclofen, bupropion, klonopin, Remeron, a GLP-1 receptor agonist, a GIP receptor agonist, or any combination thereof.

## Claims

1. A method, comprising:
maintaining, by one or more processors, for a profile associated with a user, a data structure comprising an activity field and a verification field;
determining, by the one or more processors, via one or more event handlers executing on an application, that activity data generated responsive to the user performing an activity via the application satisfies an activity condition;
updating, by the one or more processors, responsive to determining that the activity data satisfies the activity condition, the data structure to include an activity value corresponding to the activity field;
receiving, by the one or more processors, verification data associated with the user in accordance with a verification test taken by the user;
updating, by the one or more processors, responsive to determining that the verification data satisfies a verification condition, the data structure to include a verification value corresponding to the verification field; and
executing, by the one or more processors, an operation to update the profile using a token based on the activity value and the verification value.

2. The method of claim 1, further comprising generating, by the one or more processors, the token as a function of at least one of: (i) a number of updates of the data structure to include at least one activity value and at least one verification value, (ii) a time duration over which a set of activity datasets and verification datasets is received, (iii) a frequency of the updates of the data structure to include the at least one activity value and the at least one verification value over a time duration, (iv) a percentage of the at least one activity value and the at least one verification value updated in the data structure, (v) a type of activity, (vi) a type of verification test, or (vii) any combination of the foregoing, and optionally
wherein the function comprises at least one of a probabilistic function, a defined sequence, or a model.

3. The method of claim 1 or claim 2, further comprising:
determining, by the one or more processors, via the one or more event handlers executing on the application, that subsequent activity data generated responsive to the user performing a subsequent activity via the application satisfies a subsequent activity condition;
updating, by the one or more processors, responsive to determining that the subsequent activity data satisfies the subsequent activity condition, the data structure to include a subsequent activity value corresponding to a subsequent activity field;
receiving, by the one or more processors, subsequent verification data associated with the user in accordance with a subsequent verification test taken by the user;
updating, by the one or more processors, responsive to determining that the subsequent verification data satisfies a subsequent verification condition, the data structure to include a subsequent verification value corresponding to a subsequent verification field; and
executing, by the one or more processors, a subsequent operation to update the profile using a subsequent token based on the data structure.

4. The method of any preceding claim, further comprising the method further includes generating, by the one or more processors, the token as a function of at least one of: (i) a time elapsed since generation of the token, (ii) a number of tokens generated for the profile, (iii) a type of token, or (iv) a magnitude and/or probability of the token, and optionally
the method further comprising generating, by the one or more processors, a set of tokens comprising at least the token and the subsequent token using the function, responsive to executing a corresponding set of operations.

5. The method of any preceding claim, further comprising generating, by the one or more processors, the token as a set of weights corresponding to (i) a number of updates of the data structure to include at least one activity value and at least one verification value, (ii) a time duration over which a set of activity datasets and verification datasets is received, (iii) a frequency of the updates of the data structure to include the at least one activity value and the at least one verification value over a time duration, (iv) a percentage of the at least one activity value and the at least one verification value updated in the data structure, (v) a type of activity, (vi) a type of verification test, (vii) a time elapsed since generation of the token, (viii) a number of tokens generated for the profile, (ix) a type of token, (x) a magnitude and/or probability of the token, or (xi) any combination of the foregoing, and optionally
the method further comprising generating, by the one or more processors, a set of tokens using the set of weights, responsive to executing a corresponding set of operations, and optionally
wherein the method further comprises updating, by the one or more processors, at least one of the set of weights, responsive to generating at least one of the set of tokens.

6. The method of any preceding claim, further comprising:
transmitting, by the one or more processors, to a user device executing the application, an instruction to prompt the user to perform the activity via the application; and
monitoring, by the one or more processors, via the one or more event handlers, for generation of the activity data responsive to performance of the activity by the user via the application, and optionally
wherein the activity is selected from an activity related to cognitive behavioral therapy, an activity related to a psychoeducation lesson, an activity related to an assessment, an activity related to a training exercise, an activity related to a tool, an activity related to attendance, or an activity related to a condition, disease, disorder, or symptom, and optionally
the method further comprising:
determining, by the one or more processors, that the activity data generated responsive to the user performing the activity satisfies the activity condition via a computing device external to the application; and
updating, by the one or more processors, responsive to determining that the activity data satisfies the activity condition, the data structure to include the activity value corresponding to the activity field.

7. The method of any preceding claim, the method further comprising:
transmitting, by the one or more processors, to a user device executing the application, an instruction to perform the verification test based on at least one of: (i) data associated with a sample from the user, (ii) a biomarker obtained from the user, (iii) a measurement from an instrumentation device, (iv) uploading digital information to the application, (v) a verification of the user's location; or (vi) data associated with a laboratory test provided by the user or a laboratory; and
receiving, by the one or more processors, from the user device, the verification data associated with the user taking the verification test via the application.

8. The method of any preceding claim, the method further comprising receiving, by the one or more processors, from a computing device external to the application, the verification data associated with the user taking the remote test via the application in accordance with the verification test, and optionally
wherein the verification test is based on at least one of: (i) a lab test, (ii) data associated with a sample from the user, (iii) a biomarker obtained from the user, (iv) a measurement from an instrumentation device, (v) uploading digital information to the application, or (vi) a verification of the user's location.

9. The method of any preceding claim, wherein the verification test is executed to generate the verification data including a score to indicate at least one of (i) a level of substance in the user or (ii) an absence of substance in the user, and optionally
wherein the substance is selected from marijuana, cocaine, alcohol, heroine, amphetamines, opioids, nicotine, benzodiazepines, barbiturates, and metabolites thereof.

10. The method of any preceding claim, the method further comprising monitoring, by the one or more processors, during a time duration subsequent to updating the data structure to include the activity value corresponding to the activity field, for receipt of the verification data, and/or
monitoring, by the one or more processors, during a time duration subsequent to updating the data structure to include the verification value corresponding to the verification field, for receipt of the activity data, and/or
refraining, by the one or more processors, from updating the data structure to include the activity value corresponding to the activity field, responsive to determining that the activity data does not satisfy the activity condition, and/or
refraining, by the one or more processors, from updating the data structure to include the verification value corresponding to the verification field, responsive to the verification data indicating a level of substance in the user.

11. The method of any one of the proceeding claims, further comprising generating, by the one or more processors, a score indicating a number of updates to the data structure to include the activity value and the verification value, and optionally
the method further comprising providing, by the one or more processors, a graphical user interface identifying a set of scores over a set of timepoints, each score of the set of scores indicating a respective number of updates to the data structure.

12. The method of any preceding claim, further comprising:
setting, by the one or more processors, an eligibility field of the data structure to an eligibility value to enable updating of the activity field and the verification field;
providing, by the one or more processors, responsive to setting the eligibility field to the eligibility value, an instruction via the application to prompt the user to perform the activity and the verification test, and
monitoring, by the one or more processors, responsive to providing the instruction, for the activity data and the verification data, and optionally
the method further comprising setting, by the one or more processors, the eligibility field to the eligibility value, responsive to at least one of: (i) a completion of a previous activity and/or verification test, (ii) a number of completed activities and/or verification tests, or (iii) a percentage of activities and/or verification tests completed, and/or
the method further comprising identifying, by the one or more processors, the previous activity and/or verification test for which the eligibility field is to be to the eligibility value based on applying historical data to a model, and/or
the method further comprising:
generating, by the one or more processors, a score to indicate probability of enabling the updating of the activity field and the verification field; and
setting, by the one or more processors, the eligibility field of the data structure to the eligibility value, responsive to the score satisfying a threshold, and/or
the method further comprising determining, by the one or more processors, a number of times to set the eligibility field of the data structure to the eligibility value, to enable updating of the activity field and the verification field.

13. The method of any preceding claim, wherein the token is a reward to induce performance of the activity and the verification test for abstinence from a substance associated with substance abuse disorder in the user, and/or
the method further comprising executing, by the one or more processors, the operation by transferring the token to an account data structure associated with the user, and/or
the method further comprising providing, by the one or more processors, a schedule indicating a time of the activity and/or a time of the verification test, and optionally
the method further comprising providing, by the one or more processors, a notification indicating the time of the activity and/or a time of the verification test.

14. The method of any preceding claim, wherein the user is at risk of or diagnosed with substance abuse disorder, and wherein the user is taking an effective amount of a medication to address the substance abuse disorder in partial concurrence with at least one of the activity or the verification test, and optionally
wherein the medication is selected from acamprosate, naltrexone, naloxone, disulfiram, gabapentin, methadone, baclofen, bupropion, klonopin, Remeron, a GLP-1 receptor agonist, a GIP receptor agonist, or any combination thereof.

15. A system, comprising:
one or more processors coupled with memory, configured to perform the method of any preceding claim.
